# EUROPEAN PATENT SPECIFICATION

(11) **EP 3 845 152 B1**
(45) Date of publication and mention of the grant of the patent: **01.10.2025**
(21) Application number: 19857302.4
(22) Date of filing: 06.09.2019
(51) Int. Cl.: A61B 17/135, A61B 17/00, A61M 25/02, A61B 17/12

(54) **MEDICAL ADHESIVE DEVICE**
MEDIZINISCHES KLEBEVORRICHTUNG
DISPOSITIF ADHÉSIF MÉDICAL

(30) Priority: 07.09.2018 JP 2018168298
(43) Date of publication of application: 07.07.2021
(73) Proprietor: Terumo Kabushiki Kaisha, Tokyo 151-0072 (JP)
(72) Inventor: KAWAURA Masakatsu, Campbell, California 95008 (US); SOMA Yuki, Campbell, California 95008 (US); UEMURA Shuji, Mountain View, California 94040 (US); ZWEBER Matthew John, Mountain View, California 94040 (US)
(74) Representative: Casalonga
(86) International application number: PCT/JP2019/035278
(87) International publication number: WO 2020/050420

(56) References cited:
- WO-A1-2019/060229
- JP-A- 2000 500 666
- JP-A- 2001 520 089
- JP-A- 2005 521 464
- JP-A- 2015 529 115
- US-A- 3 630 195

## Description

### Technical Field

The present disclosure relates to a medical adhesion device. < - >

### Background Art

In recent years, in the medical institutions, various forms of examinations or treatments are performed using a medical insertion device which has an elongated hollow tubular shape and is called a catheter.
The catheter is percutaneously inserted into a blood vessel from a puncture site, which is formed in the wrist, the inguinal region, or the like, to be delivered to a site to be examined or treated through the blood vessel.
After the examination or treatment by a health care worker is completed, the catheter or a sheath used to introduce the catheter is extracted from the puncture site, and hemostasis is performed on the puncture site.

PTL 1 discloses a dressing as an adhesion device including a compression member that compresses a wound of a patient after a sheath is extracted.
The dressing of PTL 1 includes an inflatable bladder having a deflated state where a membrane is adjacent to an end wall and an inflated state where the membrane is spaced apart from the end wall.
In addition, the dressing of PTL 1 includes holding means that holds the bladder against the skin of the patient at a position where the wound is substantially covered.
PTL 1 discloses the holding means including a flexible web that is connected to the end wall of the bladder to protrude outward from the end wall of the bladder, and a pressure-sensitive adhesion layer which is bonded to the skin of the patient is provided on one surface as an adhesion surface of the flexible web.
During shipping and transportation, the pressure-sensitive adhesion layer is covered with a protective sheet which is removable.
The protective sheet is removed before use.

### Citation List

### Patent Literature

PTL 1: JP-T-2005-521464

### Summary of Invention

### Technical Problem

In the dressing as an adhesion device described in PTL 1, the pressure-sensitive adhesion layer provided on the one surface of the flexible web is bonded to the skin as a biological surface of the patient, and the bladder as a compression member is brought into an inflated state, so that the wound of the patient can be compressed by the bladder.

However, in the dressing described in PTL 1, when the wound of the patient is compressed by the bladder, due to a depression of the skin by compression or reaction force received from the skin, the pressure-sensitive adhesion layer provided on the one surface of the flexible web is likely to peel off in the vicinity of the bladder. When the pressure-sensitive adhesion layer peels off, a desired compression force of the bladder may not be obtained.

In addition, even an adhesion device which does not include a compression member may be likely to peel off from the skin due to external force.

Further, PTL 1 discloses the protective sheet as a release sheet which protects the pressure-sensitive adhesion layer of the adhesion surface; however, there is room for improvement in the configuration of the protective sheet.

An object of the present disclosure is to provide an adhesion device that is unlikely to peel off from a biological surface. Further, an object of the present disclosure is to provide an adhesion device including a release sheet that is easily released from an adhesion surface.

### Solution to Problem

This problem is solved by a medical adhesion device according to independent claim 1. The dependent claims relate to advantageous embodiments.

### Advantageous Effects of Invention

According to the present disclosure, it is possible to provide the adhesion device that is unlikely to peel off from the biological surface.
Further, according to the present disclosure, it is possible to provide the adhesion device including the release sheet that is easily released from the adhesion surface.

### Brief Description of Drawings

[Fig. 1] Fig. 1 is a perspective view of an adhesion device in a first embodiment.
[Fig. 2] Fig. 2 is an exploded perspective view of the adhesion device illustrated in Fig. 1.
[Fig. 3A] Fig. 3A is a side view of the adhesion device illustrated in Fig. 1.
[Fig. 3B] Fig. 3B is a side view of the adhesion device illustrated in Fig. 1 as seen in a direction different from that of Fig. 3A.
[Fig. 4A] Fig. 4A is a perspective view illustrating a state where a release sheet is removed from the adhesion device illustrated in Fig. 1.
[Fig. 4B] Fig. 4B is a top view in the state illustrated in Fig. 4A.
[Fig. 4C] Fig. 4C is a bottom view in the state illustrated in Fig. 4A.
[Fig. 5] Fig. 5 is a view illustrating the release sheet of the adhesion device illustrated in Fig. 1, and is a view illustrating a method of forming the release sheet.
[Fig. 6] Fig. 6 is a top view of the adhesion device illustrated in Fig. 1.
[Fig. 7] Fig. 7 is a bottom view of the adhesion device illustrated in Fig. 1.
[Fig. 8A] Fig. 8A is a side view in the state illustrated in Fig. 4A, and illustrates a state of a compression member before inflated.
[Fig. 8B] Fig. 8B is a side view in the state illustrated in Fig. 4A, and illustrates a state of the compression member after inflated.
[Fig. 9] Fig. 9 is a flowchart illustrating a compression method using the adhesion device illustrated in Fig. 1.
[Fig. 10A] Fig. 10A is a view illustrating an outline of an alignment step in a mounting step of Fig. 9.
[Fig. 10B] Fig. 10B is a view illustrating an outline of the alignment step in the mounting step of Fig. 9.
[Fig. 10C] Fig. 10C is a view illustrating an outline of a release sheet removing step in the mounting step of Fig. 9.
[Fig. 10D] Fig. 10D is a view illustrating an outline of the release sheet removing step in the mounting step of Fig. 9.
[Fig. 10E] Fig. 10E is a view illustrating an outline of an adhesion step in the mounting step of Fig. 9.
[Fig. 10F] Fig. 10F is a view illustrating an outline of a first compression step of Fig. 9.
[Fig. 10G] Fig. 10G is a view illustrating an outline of an extraction step of Fig. 9.
[Fig. 10H] Fig. 10H is a view illustrating an outline of a second compression step of Fig. 9.
[Fig. 11] Fig. 11 is a top view of an adhesion device in a second embodiment.
[Fig. 12] Fig. 12 is a bottom view of the adhesion device illustrated in Fig. 11.
[Fig. 13A] Fig. 13A is a side view of the adhesion device illustrated in Fig. 11.
[Fig. 13B] Fig. 13B is a side view of the adhesion device illustrated in Fig. 11 as seen in a direction different from that of Fig. 13A.
[Fig. 14] Fig. 14 is a view illustrating a release sheet of the adhesion device illustrated in Fig. 11, and is a view illustrating a method of forming the release sheet.
[Fig. 15] Fig. 15 is a perspective view of the release sheet of the adhesion device illustrated in Fig. 11.
[Fig. 16] Fig. 16 is a perspective view of an adhesion device in a third embodiment.
[Fig. 17] Fig. 17 is an exploded perspective view of the adhesion device illustrated in Fig. 16.
[Fig. 18] Fig. 18 is a top view of the adhesion device illustrated in Fig. 16.
[Fig. 19] Fig. 19 is a bottom view of the adhesion device illustrated in Fig. 16.
[Fig. 20] Fig. 20 is a view illustrating a release sheet of the adhesion device illustrated in Fig. 16, and is a view illustrating a method of forming the release sheet.
[Fig. 21] Fig. 21 is a perspective view of an adhesion device in a fourth embodiment.
[Fig. 22] Fig. 22 is an exploded perspective view of the adhesion device illustrated in Fig. 21.
[Fig. 23] Fig. 23 is a top view of the adhesion device illustrated in Fig. 21.
[Fig. 24] Fig. 24 is a bottom view of the adhesion device illustrated in Fig. 21.
[Fig. 25] Fig. 25 is a view illustrating a release sheet of the adhesion device illustrated in Fig. 21, and is a view illustrating a method of forming the release sheet.
[Fig. 26] Fig. 26 is a perspective view of an adhesion device in a fifth embodiment.
[Fig. 27] Fig. 27 is an exploded perspective view of the adhesion device illustrated in Fig. 26.
[Fig. 28] Fig. 28 is a top view of the adhesion device illustrated in Fig. 26.
[Fig. 29] Fig. 29 is a bottom view of the adhesion device illustrated in Fig. 26.
[Fig. 30] Fig. 30 is a view illustrating a release sheet of the adhesion device illustrated in Fig. 26, and is a view illustrating a method of forming the release sheet.
[Fig. 31] Fig. 31 is a description view describing when external force in a direction orthogonal to a biological surface is applied to a device main body in a state where the adhesion device illustrated in Fig. 1 adheres to the biological surface.
[Fig. 32A] Fig. 32A is a view illustrating a state where an insertion device is inserted into a femoral vein from the biological surface through a connective tissue.
[Fig. 32B] Fig. 32B is a view illustrating a state of the insertion device after extracted from the state illustrated in Fig. 32A.
[Fig. 33] Fig. 33 is a view illustrating a modification example of the release sheet illustrated in Fig. 1, and is a view illustrating a method of forming the release sheet in the modification example.
[Fig. 34] Fig. 34 is a view illustrating another modification example of the release sheet illustrated in Fig. 1, and is a view illustrating a method of forming the release sheet in the modification example.
[Fig. 35] Fig. 35 is a perspective view illustrating an adhesion device to which the release sheet illustrated in Fig. 34 adheres.

### Description of Embodiments

Hereinbelow, embodiments of an adhesion device according to the present disclosure will be described with reference to the drawings.
The same reference signs are assigned to common members and portions in the drawings.

### [First embodiment]

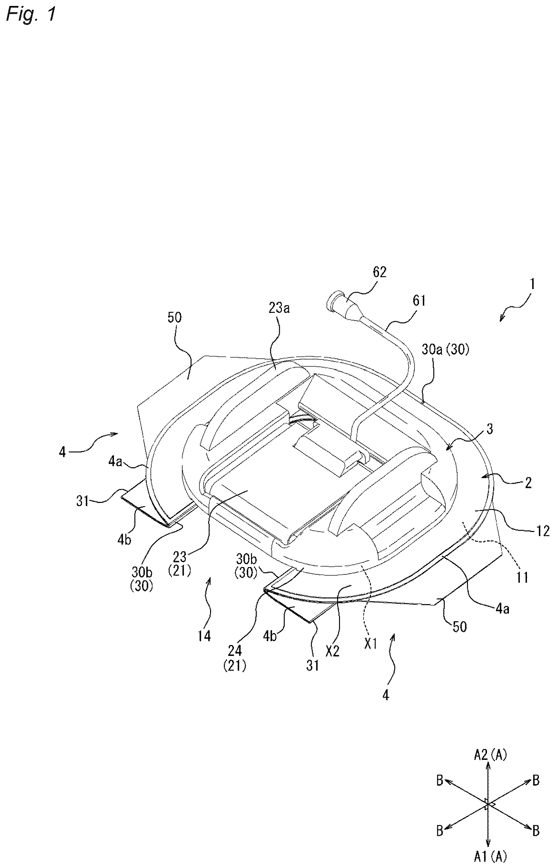

Fig. 1 is a perspective view of a medical adhesion device 1 in a first embodiment of the present disclosure.

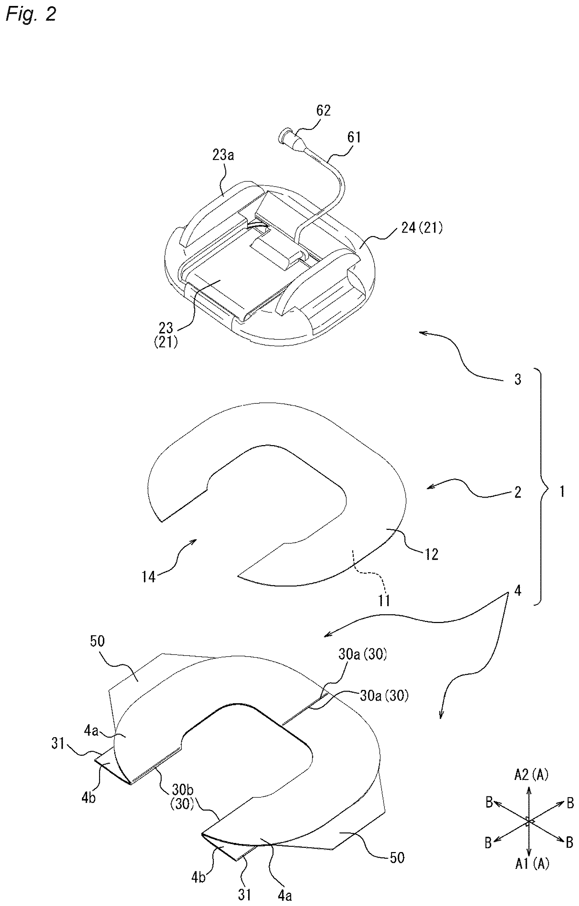

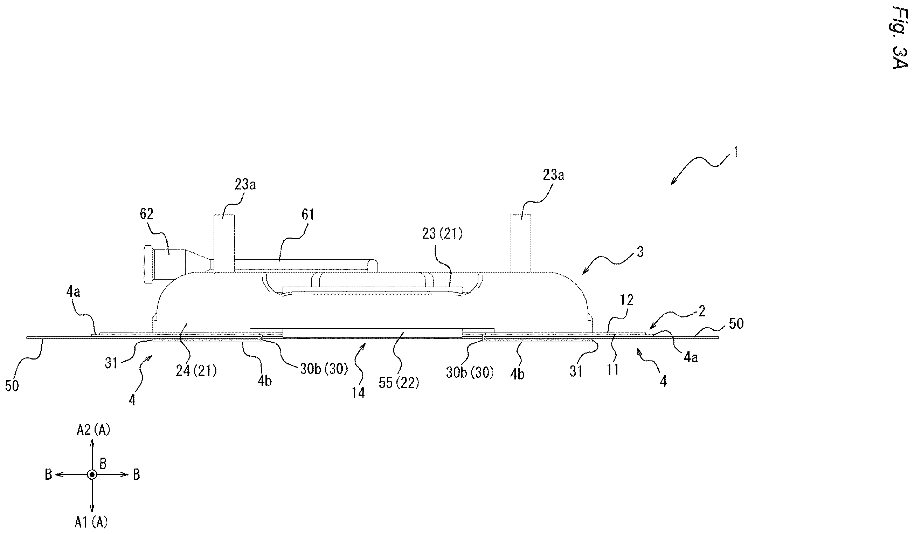

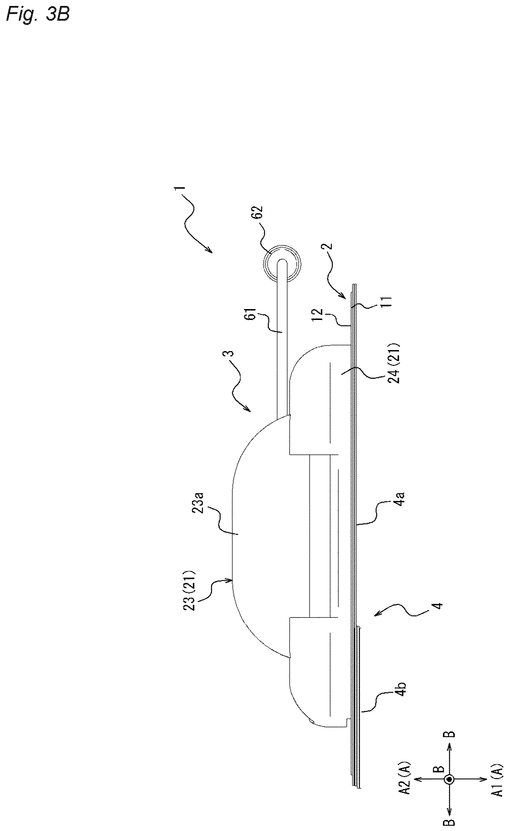

Fig. 2 is an exploded perspective view of the adhesion device 1. Figs. 3A and 3B are side views of the adhesion device 1 as seen in different directions.

As illustrated in Figs. 1 to 3, the adhesion device 1 includes an adhesion sheet 2, a device main body 3, and a release sheet 4.

The adhesion sheet 2 includes an adhesion surface 11 that is adherable to a biological surface, and a mounting surface 12 that is located on a side opposite to the adhesion surface 11.
The adhesion surface 11 is located on one side in a thickness direction A of the adhesion sheet 2.
The mounting surface 12 is located on the other side of the adhesion sheet 2 in the thickness direction A.

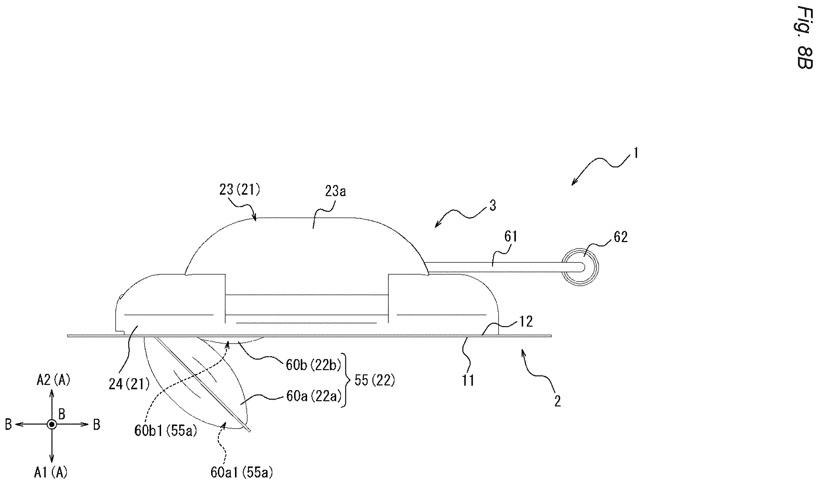

The device main body 3 is mounted to the mounting surface 12 of the adhesion sheet 2.
The configuration of the device main body 3 is not particularly limited.
The device main body 3 of the present embodiment includes a compression member 22 (refer to Fig. 8B) to be described later which can compress the biological surface, but is not limited to the configuration with the compression member 22.
Details of the compression member 22 will be described later.

The release sheet 4 releasably adheres to the adhesion surface 11 of the adhesion sheet 2.
The "adhesion" is one aspect of "mounting", and the "mounting" is not limited to adhesion.

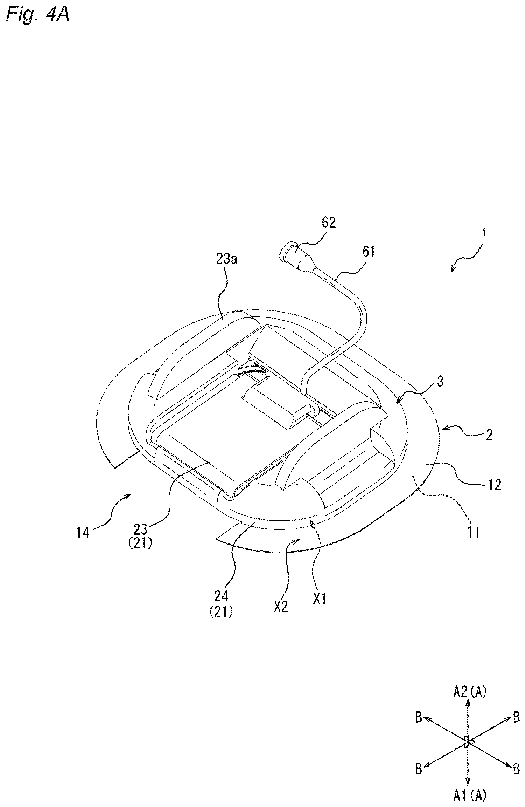

Fig. 4A is a perspective view illustrating a state where the release sheet 4 is released from the adhesion device 1 illustrated in Figs. 1 to 3.

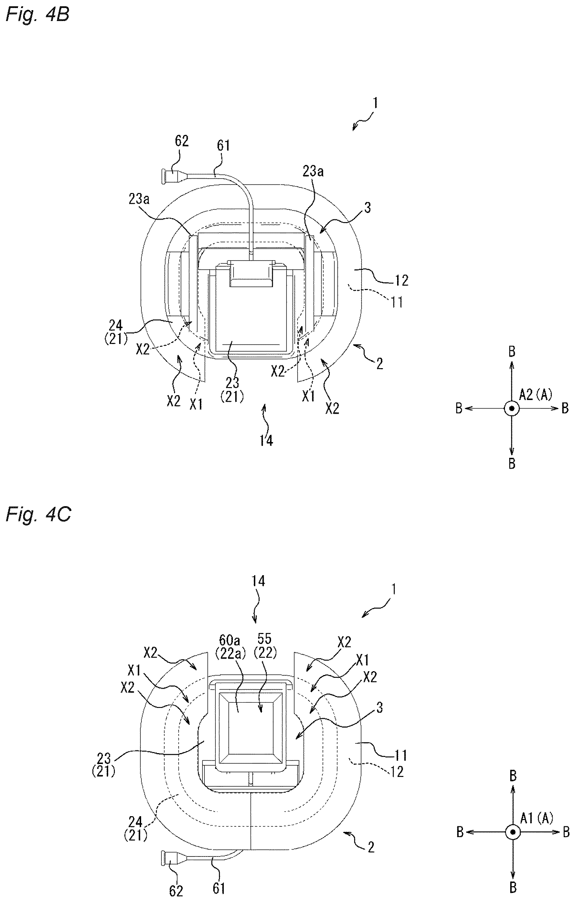

Fig. 4B is a plan view of the state illustrated in Fig. 4A as seen from a mounting surface 12 side of the adhesion sheet 2.
Fig. 4C is a plan view of the state illustrated in Fig. 4A as seen from an adhesion surface 11 side of the adhesion sheet 2.
The adhesion surface 11 adheres to the biological surface, so that the position of the adhesion device 1 on the biological surface is fixed.
When the adhesion device 1 adheres to the biological surface, the release sheet 4 is released from the adhesion surface 11.
Accordingly, the adhesion surface 11 of the adhesion sheet 2 is exposed to the outside, and the adhesion device 1 is in the state of being able to adhere to the biological surface.

Hereinafter, for convenience of description, a state where the release sheet 4 adheres to the adhesion surface 11 of the adhesion sheet 2 (refer to Figs. 1 to 3) may be simply referred to as a "pre-use state", and a state where the release sheet 4 is removed from the adhesion surface 11 of the adhesion sheet 2 (refer to Fig. 4) may be simply referred to as a "use state".

Hereinafter, for convenience of description, in the adhesion device 1, the one side in the thickness direction A, which is a direction from the mounting surface 12 toward the adhesion surface 11 in the thickness direction A of the adhesion sheet 2, is simply referred to as a "downward direction A1".
In addition, for convenience of description, in the adhesion device 1, the other side in the thickness direction A, which is a direction from the adhesion surface 11 toward the mounting surface 12 in the thickness direction A of the adhesion sheet 2, is simply referred to as an "upward direction A2".
Therefore, the adhesion surface 11 of the adhesion sheet 2 of the present embodiment is formed of a lower surface of the adhesion sheet 2.
In addition, the mounting surface 12 of the adhesion sheet 2 of the present embodiment is formed of an upper surface of the adhesion sheet 2.

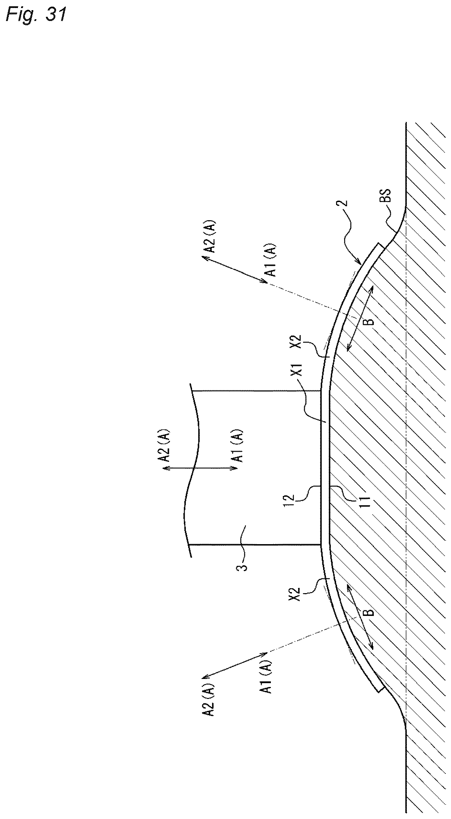

As illustrated in Figs. 4B and 4C and the like, the adhesion sheet 2 includes a first portion X1 to which the device main body 3 is mounted, and a second portion X2 to which the device main body 3 is not mounted.
The "first portion X1" means a portion of the adhesion sheet 2, which overlaps the device main body 3 in the thickness direction A, and a portion of the adhesion sheet 2, which is not separable from the device main body 3 in the thickness direction A.
In addition, the "second portion X2" means both (i) a portion of the adhesion sheet 2, which does not overlap the device main body 3 in the thickness direction A, and (ii) a portion of the adhesion sheet 2, which overlaps the device main body 3 in the thickness direction A, and a portion of the adhesion sheet 2, which is separable from the device main body 3 in the thickness direction A.
Therefore, for example, a portion of the adhesion sheet 2, which is simply covered with the device main body 3 but is separable therefrom in the thickness direction A, corresponds to the second portion X2. Although details will be described later, since the adhesion sheet 2 includes not only the first portion X1 but also the second portion X2, the adhesion device 1 can be suppressed from being unintentionally released from the biological surface (refer to Fig. 31).

In addition, as illustrated in Figs. 1 to 3, the release sheet 4 includes a release layer 4a that adheres to the adhesion surface 11 of the adhesion sheet 2, and a folded layer 4b which is formed of at least one layer (in the present embodiment, a part is in three layers and the other part is in one layer) and is folded to a side, which is opposite to the adhesion surface 11 with respect to the release layer 4a, to be layered.

As illustrated in Fig. 1, the folded layer 4b of the release sheet 4 is layered on the release layer 4a of the release sheet 4 at least at the position of the second portion X2 of the adhesion sheet 2.
Since the release sheet 4 has such a configuration, the release sheet 4 is easily released from the second portion X2 of the adhesion sheet 2.
The release sheet 4 can be released from the adhesion sheet 2 by moving the folded layer 4b in an extending direction B of the adhesion sheet 2, which is orthogonal to the thickness direction A of the adhesion sheet 2.
For this reason, as compared to a configuration without the folded layer 4b, when the release sheet 4 is released from the adhesion sheet 2, it is more difficult to apply force to the adhesion sheet 2 in the thickness direction A which is an out-of-plane direction of the adhesion sheet 2.
On the other hand, it is easy to apply force to the adhesion sheet 2 in the extending direction B which is an in-plane direction of the adhesion sheet 2.
Accordingly, when the release sheet 4 is released from the adhesion sheet 2, the second portion X2 of the adhesion sheet 2, which is not mounted to the device main body 3, can be suppressed from being bent together with the release sheet 4.

Therefore, since the adhesion sheet 2 includes the second portion X2 as described above, the adhesion device 1 is unlikely to peel off from the biological surface.
Further, in the adhesion device 1, the release sheet 4 includes the folded layer 4b in the second portion X2 of the adhesion sheet 2.
For this reason, the release sheet 4 is easily released from the adhesion surface 11 even in the second portion X2 of the adhesion sheet 2.

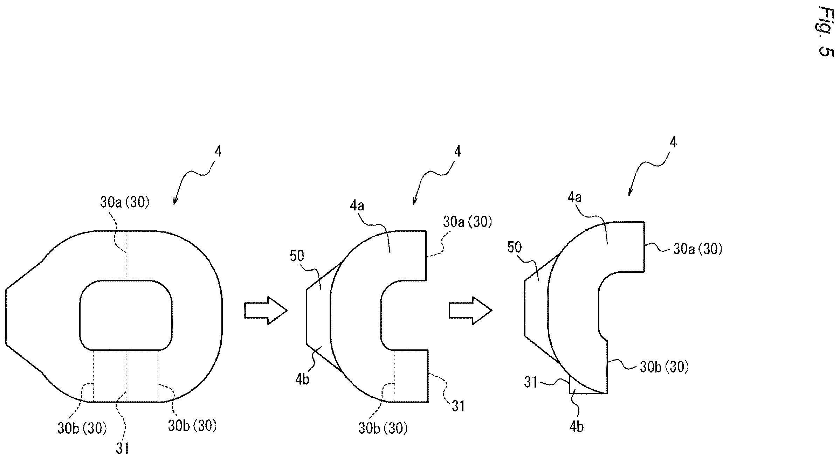

Fig. 5 is a view illustrating the release sheet 4 of the adhesion device 1.
Fig. 5 illustrates a method of forming the release sheet 4.
As illustrated in Fig. 2 and the like, in the present embodiment, the entire region of the adhesion surface 11 of the adhesion sheet 2 is covered with two release sheets 4 which are symmetrical in shape.
Fig. 5 illustrates only one release sheet 4 of the two release sheets 4 which are symmetrical in shape.
Since the two release sheets 4 are symmetrical in shape, hereinafter, only the one release sheet 4 will be described as an example.

As illustrated in Fig. 5, the release sheet 4 of the present embodiment is folded once from an original form of a single layer (left figure in Fig. 5) to be shaped into an intermediate form of two layers (middle figure in Fig. 5).
Further, the release sheet 4 of the present embodiment is further partially folded from the intermediate form of two layers to be shaped into a completed form (right figure in Fig. 5) in which a part is in four layers and the other part is in two layers.
In such a manner, the release sheet 4 including the release layer 4a which is formed of one layer and the folded layer 4b of which a part is formed of three layers and the other part is formed of one layer is formed.

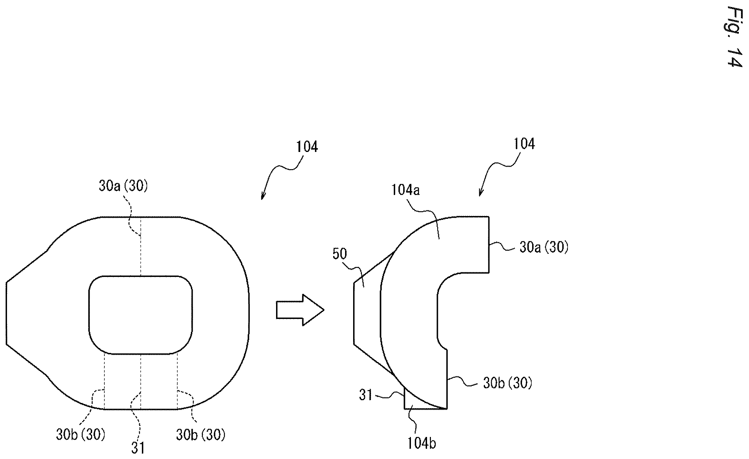

The configuration of the release layer 4a and the folded layer 4b of the release sheet 4 is not limited to the configuration illustrated in Fig. 5.
In the present embodiment, the release sheet 4 is used in the completed form illustrated in Fig. 5 (right figure in Fig. 5); however, for example, the release sheet may be used in the intermediate form illustrated in Fig. 5 (middle figure in Fig. 5).
In the present embodiment, the release sheet 4 is partially folded from the intermediate form illustrated in Fig. 5 to be shaped into the completed form, so that a receiving portion 14 (refer to Fig. 1 and the like) which can receive an insertion device such as a sheath is secured.
Details of the receiving portion 14 will be described later.
In addition, examples of release sheets having shapes different from that of the release sheet 4 of the present embodiment will be also described later (refer to Figs. 14, 15, 20, 25, and 30).

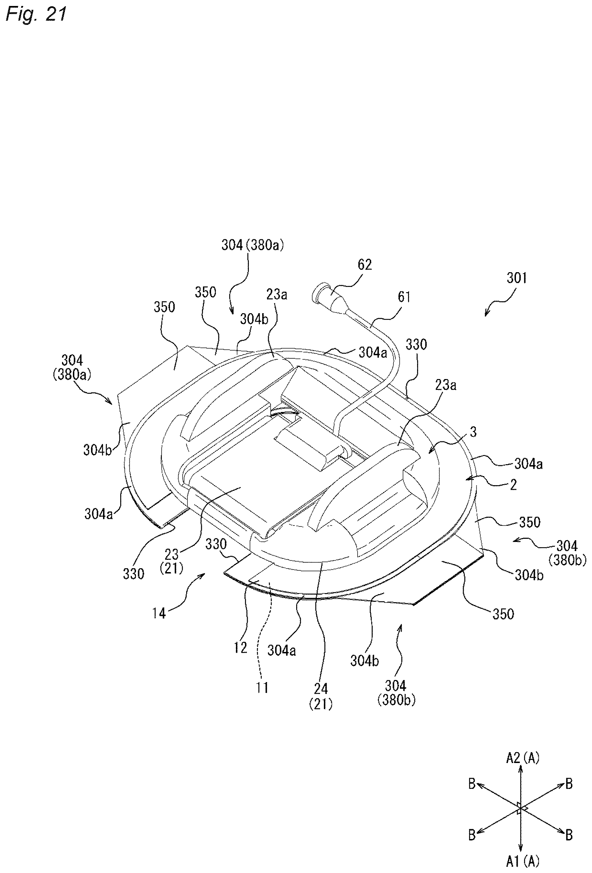

Further, in the present embodiment, as illustrated in Fig. 2 and the like, the two release sheets 4 are mounted to the adhesion surface 11 of one adhesion sheet 2; however, the entire region of the adhesion surface 11 of the adhesion sheet 2 may be covered with one release sheet, or may be covered with three or more release sheets (refer to Figs. 21 to 25).

As described above, the folded layer 4b of the release sheet 4 forms a sheet grip portion that is gripped when the release sheet 4 is released from the adhesion sheet 2.
The folded layer 4b as the sheet grip portion may include only a portion that overlaps the release layer 4a in the thickness direction **A.**
Meanwhile, it is preferable that the sheet grip portion includes a designated grip portion.
As illustrated in Fig. 1 and the like, the sheet grip portion of the present embodiment includes an extending portion 50 that is formed of a portion of the folded layer 4b of the release sheet 4, the portion not overlapping the release layer 4a in a plan view seen along the thickness direction A of the adhesion sheet 2.
The designated grip portion of the sheet grip portion of the present embodiment is formed of the extending portion 50.
Since the sheet grip portion includes the extending portion 50 as a designated grip portion, the operability of releasing the release sheet 4 from the adhesion sheet 2 can be improved.
The extending portion 50 of the present embodiment is located outside the adhesion sheet 2 and the device main body 3 in a plan view seen along the thickness direction A of the adhesion sheet 2.
Further, the extending portion 50 of the present embodiment is located outside the release layer 4a in a plan view seen along the thickness direction A of the adhesion sheet 2.

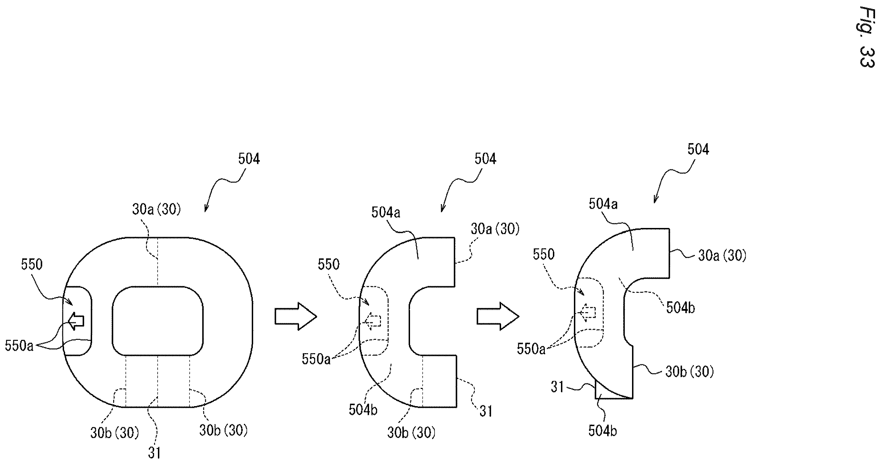

In addition, Fig. 33 is a view illustrating a release sheet 504 as a modification example of the release sheet 4 illustrated in Figs. 1 to 5.
Fig. 33 illustrates a method of forming the release sheet 504.
A folded layer 504b of the release sheet 504 illustrated in Fig. 33 includes a grip guide portion 550 which is a portion overlapping a release layer 504a in a plan view seen along the thickness direction A of the adhesion sheet 504, and to which a predetermined mark 550a which provides a guide on gripping is applied.
In the example illustrated in Fig. 33, a designated grip portion of the sheet grip portion is formed of the grip guide portion 550.
Examples of the mark 550a include various symbols, patterns, characters, combinations thereof, and the like which guide that the grip guide portion 550 is to be gripped to release the release sheet 504.
In addition, the mark 550a may include various symbols such as arrows, characters, and the like that also guide a direction in which the grip guide portion 550 is to be moved when the release sheet 504 is released, in addition to simply providing a guide on gripping.
The grip guide portion 550 illustrated in Fig. 33 provides a guide on gripping with a line indicating a grip range, and guides the direction, in which the grip guide portion 550 is to be moved when the release sheet 504 is released, with an arrow.
Namely, the mark 550a which is formed of a line and an arrow is applied to the grip guide portion 550 illustrated in Fig. 33.

As described above, the designated grip portion of the sheet grip portion may be the extending portion 50 described above (refer to Fig. 5 and the like), or may be the grip guide portion 550 (refer to Fig. 33).
In Fig. 33, the extending portion is not provided; however, a configuration in which the extending portion is provided in addition to the grip guide portion 550 may be employed.
In addition, in Fig. 5, the grip guide portion is not provided; however, a configuration in which the grip guide portion is provided in addition to the extending portion 50 may be employed.
In addition, a mark which provides a guide on gripping or a mark which guides the movement direction when the release sheet 4 is released may be applied to the extending portion 50 as the designated grip portion illustrated in Figs. 1 to 5.

Hereinafter, each member and portion of the adhesion device 1 of the present embodiment will be described in further detail.

### <Adhesion sheet 2>

The adhesion sheet 2 is flexible.
Namely, the adhesion sheet 2 is easily deformable in the out-of-plane direction.

For this reason, the adhesion sheet 2 is deformable along the shape of the biological surface.

In addition, since the adhesion surface 11 adheres to the biological surface, the adhesion sheet 2 is easily deformed according to deformation of the biological surface.

As a result, the adhesion surface 11 is unlikely to peel off from the biological surface, and the adhesion device 1 can be suppressed from being unintentionally released from the biological surface.

Means for mounting the device main body 3 to the mounting surface 12 of the adhesion sheet 2 is not particularly limited.
The device main body 3 and the mounting surface 12 of the adhesion sheet 2 may be joined, for example, by bonding using a bonding agent, fusion, adhesion, or the like.
The release strength of the device main body 3 with respect to the mounting surface 12 of the adhesion sheet 2 is larger than at least the release strength of the release sheet 4 with respect to the adhesion surface 11 of the adhesion sheet 2.
The "release strength" referred to here can be measured, for example, by a 180° release test of the adhesion sheet 2.

The adhesion sheet 2 includes a plurality of layers including, for example, a base material layer and a bonding layer.

The base material layer is made of, for example, a thin resin sheet.
More specifically, the base material layer of the present embodiment is made of a white spunlace non-woven fabric of polyester fibers, and the thickness of the base material layer is in a range of 5 µm to 150 µm, for example, 30 µm or the like.
Meanwhile, the material of the base material layer is not limited to polyester, and for example, acrylic polymer, polyethylene, ethylene-vinyl acetate copolymer, polyurethane, polyamide derivative, or the like may be used.

The bonding layer is formed of a bonding agent such as a rubber-based bonding agent, an acrylic-based bonding agent, a silicon-based bonding agent, or the like.
The bonding layer is laminated on the base material layer directly or indirectly with another layer interposed therebetween.
The adhesion surface 11 of the adhesion sheet 2 of the present embodiment is formed of the bonding layer.

As described above, the adhesion sheet 2 of the present embodiment is made of a non-woven fabric tape, to one surface of which a pressure-sensitive adhesive is applied as a bonding agent, but may be made of a double sided tape in which bonding layers are provided on both sides of a base material layer.
When the adhesion sheet is made of a double sided tape, the device main body 3 is bonded to one bonding layer of the adhesion sheet, so that the device main body 3 can be fixed to the adhesion sheet.

The adhesion sheet 2 is not limited to a structure of two layers including the base material layer and the bonding layer, and may have, for example, a structure of three or more layers further including another layer.
The adhesion sheet may include, for example, a base material layer, a bonding layer, and a surface layer.
For example, the surface layer is laminated, directly or indirectly with another layer interposed between the surface layer and the base layer, on an opposite side of the base material layer from the bonding layer with the base material layer interposed therebetween.
Namely, the surface layer forms a mounting surface of the adhesion sheet.
Since such a surface layer is provided, the mechanical strength can be adjusted.
For example, the surface layer may be bonded with another bonding layer intervening between the base material layer and the surface layer.
The surface layer can be made of, for example, a resin having a thickness of approximately 5 µm to 50 µm.
More specifically, as the material of the surface layer, polyester, polyamide, polyamideimide, polyethylene, polypropylene, polycarbonate, polyurethane, polyvinyl chloride, fluororesin, and the like can be used.

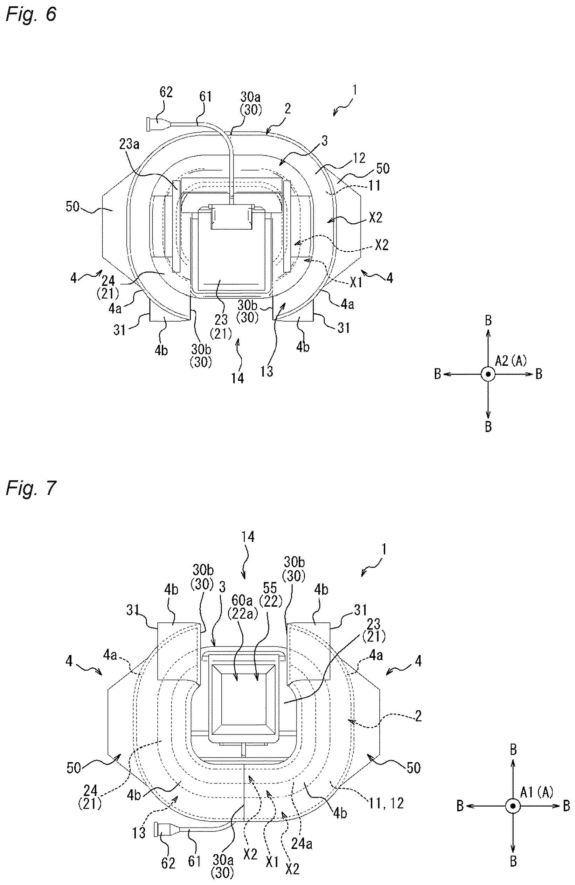

Figs. 6 and 7 are plan views of the adhesion device 1 as seen along the thickness direction A of the adhesion sheet 2.
More specifically, Fig. 6 is a plan view of the adhesion device 1 as seen from the mounting surface 12 side of the adhesion sheet 2, to which the device main body 3 is mounted.
Fig. 7 is a plan view of the adhesion device 1 as seen from the adhesion surface 11 side of the adhesion sheet 2, to which the release sheet 4 is mounted.
Hereinafter, for convenience of description, a plan view of the adhesion device 1 seen along the thickness direction A of the adhesion sheet 2 (refer to Figs. 4B, 4C, 6, and 7) is simply referred to as a "plan view".
In addition, among plan views of the adhesion device 1, a plan view of the adhesion device 1 seen from the mounting surface 12 side of the adhesion sheet 2 (refer to Figs. 4B and 6) may be simply referred to as a "top view".
In addition, among the plan views of the adhesion device 1, a plan view of the adhesion device 1 seen from the adhesion surface 11 side of the adhesion sheet 2 (refer to Figs. 4C and 7) may be simply referred to as a "bottom view".

As illustrated in Fig. 4B and the like, in a plan view, the adhesion sheet 2 extends in a C shape.
In other words, in a plan view, the adhesion sheet 2 of the present embodiment defines a central opening region.
An open region of the central opening region forms the receiving portion 14 to be described later, the open region communicating with the outside.
The open region in the central opening region is a region that is interposed between both end portions of the adhesion sheet 2 having a C shape in a plan view.

As described above, the outer shape of the adhesion sheet 2 of the present embodiment is a C shape in a plan view, but is not limited to the shape. For example, an adhesion sheet having an endless outer shape in a plan view may be used.
The outer edge shape and the inner edge shape of the adhesion sheet having an endless outer shape in a plan view are not particularly limited.
Various shapes such as polygonal shapes including a circular shape, an elliptical shape, and a quadrilateral shape can be employed.

In addition, it is preferable that a slit extending from an outer edge to an inner edge is formed in the adhesion sheet having an endless outer shape in a plan view.
Since such a slit is provided, the central opening region and the outside communicate with each other through the slit.
For this reason, the insertion device such as a catheter or a sheath can be moved from outside the adhesion sheet to the central opening region through the slit.
As described above, the slit may form the receiving portion 14.

Next, the first portion X1 and the second portion X2 of the adhesion sheet 2 will be described.

As described above, the adhesion sheet 2 includes the first portion X1 to which the device main body 3 is mounted, and the second portion X2 to which the device main body 3 is not mounted (refer to Fig. 1 and the like).
More specifically, the adhesion sheet 2 includes the second portion X2 that extends continuously from the first portion X1.
An adhesion portion formed of a bonding agent or the like is provided in at least a part of the adhesion surface 11 at a position corresponding to the second portion X2.

Since the adhesion sheet 2 of the adhesion device 1 includes the second portion X2 described above, the adhesion sheet 2 can be suppressed from unintentionally peeling off from the biological surface.
Hereinafter, the details will be described.

The device main body 3 is mounted to the mounting surface 12 of the adhesion sheet 2.
For this reason, the adhesion device 1 is likely to receive external force even in a state where the adhesion device 1 adheres to the biological surface.
Fig. 31 illustrates a state where external force in a direction orthogonal to a biological surface BS is applied to the device main body 3 in a state where the adhesion surface 11 of the adhesion sheet 2 adheres to the biological surface BS.
As illustrated in Fig. 31, when the external force in the direction orthogonal to the biological surface BS is applied to the device main body 3, the first portion X1 of the adhesion sheet 2 to which the device main body 3 is mounted is lifted in a direction away from the biological surface BS.
Accordingly, a portion of the biological surface BS, to which the first portion X1 adheres, is deformed to be lifted according to the first portion X1.
Even when the above-described deformation occurs in the biological surface BS, the second portion X2 extending from the first portion X1 maintains the state of adhesion according to the biological surface BS.
For this reason, force in a direction along a raised portion of the adhesion surface 11 and the biological surface BS is likely to be applied to the second portion X2 extending from the first portion X1.

Namely, the application of the force in the direction orthogonal to the biological surface to the second portion X2 extending from the first portion X1 can be reduced.
In addition, shearing force in a direction along the biological surface BS is likely to be applied to the second portion X2 of the adhesion sheet 2.
The adhesion sheet 2 is unlikely to be moved and peeled off by the shearing force in the direction along the biological surface BS.
As described above, since the adhesion sheet 2 is provided with the second portion X2, it is possible to realize the adhesion sheet 2 which is more unlikely to peel off from the biological surface BS as compared to a configuration without the second portion X2.

Particularly, as in the present embodiment, when the adhesion device 1 is a compression device, it is preferable that the adhesion sheet 2 includes not only the first portion X1 but also the second portion X2.
Although details will be described later, the adhesion device 1 of the present embodiment is a compression device including the compression member 22 (refer to Fig. 8B).
Namely, in the adhesion device 1 of the present embodiment, the compression member 22 can compress a predetermined site on the biological surface in a state where the adhesion surface 11 adheres to the biological surface.
Examples of the predetermined site on the biological surface, which is compressed by the adhesion device 1, include a wound and the vicinity of the wound formed by insertion of a medical insertion device such as a puncture needle, a catheter, or a sheath into a blood vessel of a living body.
After the above-described insertion device such as a sheath is extracted from the living body, the wound or the vicinity of the wound on the biological surface is compressed by the compression member 22 of the adhesion device 1, so that hemostasis can be achieved.
Since the adhesion surface 11 of the adhesion sheet 2 adheres to the biological surface, the device main body 3 including the compression member 22 can maintain a state where the biological surface is compressed.
In other words, a compression force from the compression member 22 to the biological surface is applied at the position of the first portion X1 of the adhesion sheet 2 as a force to lift the adhesion surface 11 to the upward direction A2 in the thickness direction A.
Namely, as illustrated in Fig. 31, a force to pull the first portion X1 of the adhesion sheet 2 in a direction away from the biological surface BS is likely to be generated.
For this reason, when the adhesion device 1 is a compression device, it is particularly preferable that the adhesion sheet 2 includes not only the first portion X1 but also the second portion X2.

Further, as illustrated in Fig. 7, it is preferable that the second portion X2 of the adhesion sheet 2 is provided on at least a compression member 22 side with respect to the first portion X1.
A compression member 22 side of the first portion X1 of the adhesion sheet 2 is more likely to be released due to the compression force of the compression member 22 as compared to a side opposite to the compression member 22 side.
For this reason, the second portion X2 is provided on at least the compression member 22 side with respect to the first portion X1, so that release of the adhesion sheet 2 can be more reliably suppressed.

Meanwhile, as illustrated in Fig. 7, it is preferable that the second portion X2 of the adhesion sheet 2 is provided not only on the compression member 22 side with respect to the first portion X1 but also on the side opposite to the compression member 22 side.
As a consequence, release of the adhesion sheet 2 can be even further suppressed.

Further, as illustrated in Fig. 6 and the like, the adhesion sheet 2 of the present embodiment includes an outer peripheral portion 13 that is located on a periphery outside the device main body 3 in a plan view.
In other words, the outer peripheral portion 13 of the adhesion sheet 2 of the present embodiment is the second portion X2 that is not mounted to the device main body 3.

As illustrated in Fig. 6, in the present embodiment, in a plan view, the outer peripheral portion 13 of the adhesion sheet 2 does not fully surround the periphery of the device main body 3.
Namely, a position where the outer peripheral portion 13 of the adhesion sheet 2 is not provided exists around the device main body 3.
A portion of the insertion device such as a sheath which is inserted into the living body is received at the position where the outer peripheral portion 13 of the adhesion sheet 2 is not provided, the portion extending outside the living body.
Namely, the receiving portion 14 which can receive the insertion device such as a sheath is defined around the device main body 3 at the position where the outer peripheral portion 13 of the adhesion sheet 2 is not provided.
Accordingly, the compression member 22 of the device main body 3 can be brought close to the wound or the vicinity of the wound on the biological surface, the wound being formed by the insertion device such as a sheath.
For this reason, after the insertion device such as a sheath is extracted from the living body, the wound or the vicinity of the wound on the biological surface is easily compressed by the compression member 22 of the device main body 3 of the adhesion device 1.

### <Device main body 3>

As described above, the adhesion device 1 of the present embodiment is a compression device.
Here, the device main body 3 including the compression member 22 will be described as an example; however, a device main body without the compression member 22 may be used.

As illustrated in Fig. 4B and the like, in a plan view, the device main body 3 of the present embodiment is disposed across the contour of the adhesion sheet 2.
In other words, in a plan view, the device main body 3 includes a portion that overlaps the adhesion sheet 2, and a portion that does not overlap the adhesion sheet 2.
More specifically, in a plan view, the device main body 3 of the present embodiment is disposed to cover the central opening region of the adhesion sheet 2.
The device main body 3 of the present embodiment is fixed to the mounting surface 12 of the adhesion sheet 2 at the position of an outer edge portion of the device main body 3.
In the device main body 3 of the present embodiment, the compression member 22 to be described later can compress the biological surface in the central opening region defined by the adhesion sheet 2.

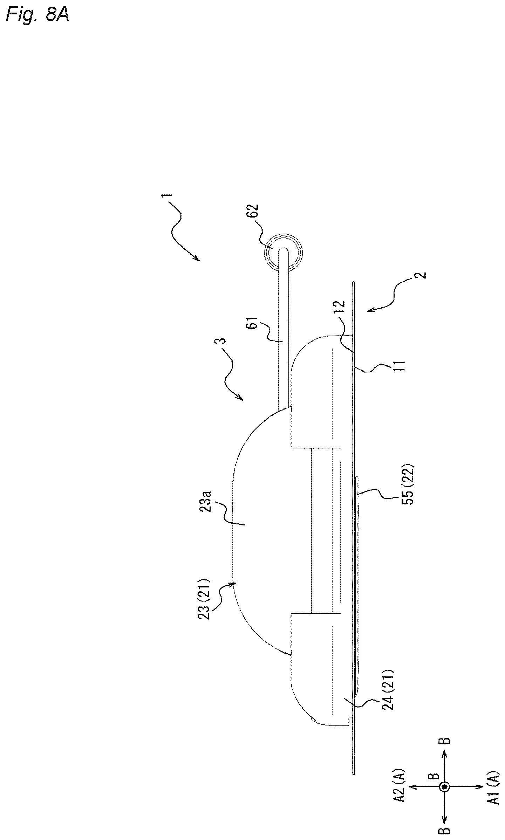

Figs. 8A and 8B are side views of the adhesion device 1 in a use state.
The adhesion device 1 in a use state illustrated in Figs. 8A and 8B is in a side view seen from the same viewpoint as that of the side view of the adhesion device 1 in a pre-use state illustrated in Fig. 3B.
Fig. 8A illustrates a state of an inflator 55 as the compression member 22 to be described later before inflated.
Fig. 8B illustrates a state of the inflator 55 as the compression member 22 after inflated.

As illustrated in Figs. 8A and 8B, the device main body 3 of the present embodiment includes a holder 21 and the compression member 22.

### <<Holder 21>>

The holder 21 is fixed to the mounting surface 12 of the adhesion sheet 2.
In addition, the holder 21 holds the compression member 22.

As illustrated in Fig. 4B and the like, in a plan view, the holder 21 of the present embodiment includes a portion that overlaps the adhesion sheet 2, and a portion that does not overlap the adhesion sheet 2.
The portion of the holder 21 of the present embodiment, which overlaps the adhesion sheet 2, is fixed to the mounting surface 12 of the adhesion sheet 2.
The portion of the holder 21 of the present embodiment, which does not overlap the adhesion sheet 2, is located in the central opening region defined by the adhesion sheet 2 in a plan view.

Examples of the material of the holder 21 include a resin material.
Examples of the resin material include thermoplastic resins used in injection molding, such as ABS resin, AS resin, polyethylene, polypropylene, polystyrene, polyvinyl chloride, polyvinylidene chloride resin, polyphenylene oxide, thermoplastic polyurethane, polymethylene methacrylate, polyoxyethylene, fluororesin, polycarbonate, polyamide, acetal resin, acrylic resin, and polyethylene terephthalate, thermosetting resins such as phenol resin, epoxy resin, silicone resin, and unsaturated polyester, and the like.

### <<Compression member 22>>

The compression member 22 is held by the holder 21.
The compression member 22 is protrudable toward the downward direction A1, which is the one side in the thickness direction A, from the adhesion surface 11 of the adhesion sheet 2.
In the adhesion device 1, in a state where the adhesion surface 11 of the adhesion sheet 2 adheres to the biological surface, the compression member 22 protrudes from the adhesion surface 11 of the adhesion sheet 2 to the downward direction A1.
Accordingly, the biological surface is compressed by the compression member 22.

The compression member 22 of the present embodiment is the inflator 55 that is inflatable toward the downward direction A1 in the thickness direction A by supply of a fluid such as air.
The form of the inflator 55 of the present embodiment is changed from a retracted form illustrated in Fig. 8A to a protruding form illustrated in Fig. 8B, so that the inflator 55 protrudes from the adhesion surface 11 of the adhesion sheet 2 toward the downward direction A1 in the thickness direction A to assume a posture, thus to be able to compress the biological surface.

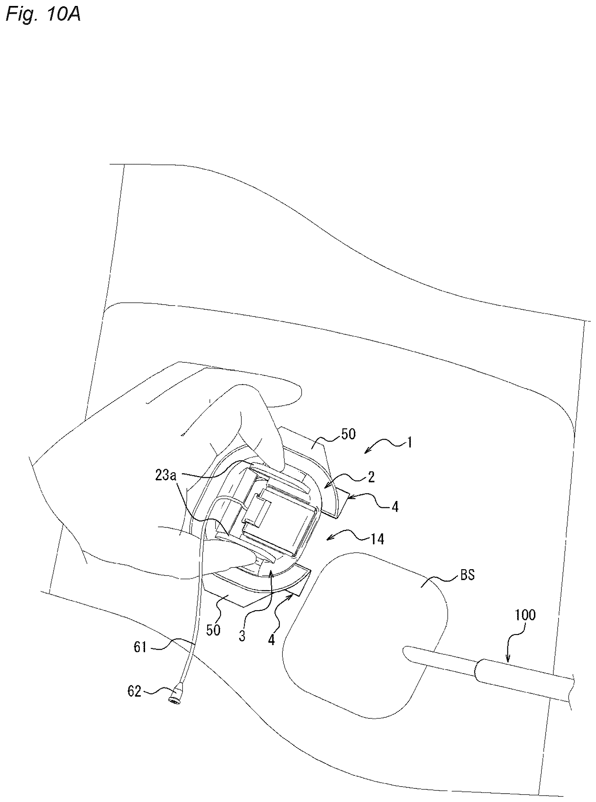

Specifically, the adhesion device 1 of the present embodiment adheres to the biological surface when the adhesion device is in the retracted form (refer to Fig. 8A) before the inflator 55 as the compression member 22 is inflated.
In a state where the adhesion device 1 is fixed to the biological surface, the inflator 55 is inflated by an operation by an operator such as a health care worker, to assume the protruding form (refer to Fig. 8B).
Accordingly, in the adhesion device 1 of the present embodiment, the inflator 55 can compress a predetermined site on the biological surface.
As described above, the predetermined site on the biological surface is a wound or the like formed by insertion of the medical insertion device into a blood vessel of the living body.
After the above-described insertion device such as a sheath is extracted from the living body, the wound or the vicinity of the wound on the biological surface is compressed by the compression member 22 of the adhesion device 1, so that hemostasis can be achieved.
Details of a method of the series of compression will be described later (refer to Figs. 10A to 10H).

As illustrated in Fig. 8A, the inflator 55 as the compression member 22 of the present embodiment in the retracted form is substantially flush with the adhesion surface 11 of the adhesion sheet 2, and does not protrude from the adhesion surface 11 to the downward direction A1.
The holder 21 may include a lock mechanism which performs locking such that the inflator 55 in the retracted form does not protrude from the adhesion surface 11 of the adhesion sheet 2 to the downward direction A1.

The inflator 55 of the present embodiment defines an internal space 55a.
The internal space 55a of the inflator 55 communicates with a tube 61 that penetrates through the holder 21 to extend outside the holder 21.
For example, a fluid such as air is supplied through the tube 61 to the internal space 55a of the inflator 55 from a fluid supply device such as a syringe that is connected to an inflation port as a connection section 62 provided in an end portion of the tube 61.
Accordingly, the form of the inflator 55 can be changed from the retracted form (refer to Fig. 8A) to the protruding form (refer to Fig. 8B).
Namely, the inflator 55 as the compression member 22 is protrudable to the downward direction A1 from the adhesion surface 11 of the adhesion sheet 2.
The fluid to be supplied to the internal space of the inflator 55 is not limited to a gas, and may be a liquid.
In addition, when the fluid is aspirated from the internal space 55a of the inflator 55, the form of the inflator 55 can be changed from the protruding form (refer to Fig. 8B) to the retracted form (refer to Fig. 8A).

More specifically, as illustrated in Fig. 8B, the compression member 22 of the present embodiment includes a contact portion 22a that comes into contact with the biological surface to directly compress the biological surface, and a pressing portion 22b that presses the contact portion 22a in a predetermined direction to increase the compression force of the contact portion 22a in the predetermined direction.
Specifically, the inflator 55 as the compression member 22 of the present embodiment includes a first inflator 60a as the contact portion 22a and a second inflator 60b as the pressing portion 22b.

The first inflator 60a defines a first internal space 60a1.
The second inflator 60b defines a second internal space 60b1.
The first internal space 60a1 and the second internal space 60b1 communicate with each other.
In the retracted form of the inflator 55, the first inflator 60a and the second inflator 60b in a deflated state overlap each other in the thickness direction A.
The first inflator 60a in a deflated state is located on a lower side, and the second inflator 60b in a deflated state is located on an upper side to overlap the first inflator 60a.
The upward direction A2 of the second inflator 60b is covered with the holder 21.
In addition, the first inflator 60a and the second inflator 60b are continuous with each other only at one end (left side in Fig. 8B) in a direction orthogonal to the thickness direction A.

The fluid which is supplied to the internal space 55a of the inflator 55 through the tube 61 flows into the second internal space 60b1 of the second inflator 60b, and then flows into the first internal space 60a1 of the first inflator 60a.
For this reason, when the fluid is supplied to the internal space 55a of the inflator 55, first, the second inflator 60b is inflated.
As described above, since the upward direction A2 of the second inflator 60b is covered with the holder 21, the second inflator 60b to which the fluid has been supplied is inflated toward the downward direction A1.
Next, the fluid flows into the first internal space 60a1, and the first inflator 60a is inflated.
At that time, the first inflator 60a is inflated to move rotationally around the one end (left side in Fig. 8B) in the direction orthogonal to the thickness direction A, the second inflator 60b being continuous with the first inflator 60a in the one end as a central axis of the rotary movement.
Namely, when the fluid is supplied to the first internal space 60a1 of the first inflator 60a, since the first inflator 60a is pressed by the second inflator 60b, the first inflator 60a is inflated such that the other end (right side in Fig. 8B) with respect to the one end in the direction orthogonal to the thickness direction A moves to the downward direction A1, to assume the protruding form illustrated in Fig. 8B.
Accordingly, the compression force of the first inflator 60a in the predetermined direction (compression force in a lower left direction in Fig. 8B) can be increased.

As the inflator 55, a balloon which is inflated by, for example, a gas such as air can be used.
As the forming material of the inflator 55, a material which is flexible such as soft polyvinyl chloride, polyurethane, polyethylene, polypropylene, polyester, ethylene-vinyl acetate copolymer (EVA), silicone, or a mixture of any of these materials can be used.

The configuration of the compression member 22 is not particularly limited.
A compression member without the pressing portion 22b may be employed.
In addition, even when the compression member 22 is formed of an inflator, the configuration is not limited to the above-described configuration of the inflator 55.
Further, the compression member 22 of the present embodiment is configured to protrude from the adhesion surface 11 of the adhesion sheet 2 to the downward direction A1 by inflation, but may be a compression member that normally protrudes from the adhesion surface 11 of the adhesion sheet 2 to the downward direction A1.

In addition, as illustrated in Fig. 8B, when the compression member 22 includes the pressing portion 22b, it is preferable that the contact portion 22a of the compression member 22 is disposed to be biased toward a pressing direction (left side in Fig. 8B), in which the pressing portion 22b performs pressing, in the holder 21. In other words, as illustrated in Fig. 7, it is preferable that the contact portion 22a of the compression member 22 is disposed to be biased toward a receiving portion 14 side of the adhesion sheet 2 from the center position of the holder 21 in the holder 21 in a plan view.

### <<<Cover portion 23 of holder 21>>>

Next, the holder 21 of the present embodiment will be described in further detail.
The holder 21 of the embodiment includes a cover portion 23 and a support portion 24.

The cover portion 23 covers the upward direction A2 of the inflator 55 as the compression member 22.
In addition, the cover portion 23 is mounted to the inflator 55 as the compression member 22.

As illustrated in Fig. 4B and the like, in a plan view, the cover portion 23 of the present embodiment is located in the central opening region defined by the adhesion sheet 2.
In other words, the cover portion 23 of the present embodiment is a portion of the holder 21, which does not overlap the adhesion sheet 2 in a plan view.

As illustrated in Fig. 1, the cover portion 23 of the present embodiment includes two plate portions 23a that protrude upward.
The two plate portions 23a are disposed to face each other, and form a grip portion of the device main body 3.
In the present embodiment, the two plate portions 23a form the grip portion; however, the grip portion is not limited to the configuration.
The grip portion may be formed of one plate portion 23a.

### <<<Support portion 24 of holder 21>>>

The support portion 24 supports the cover portion 23, and is fixed to the mounting surface 12 of the adhesion sheet 2.
In addition, a part of the support portion 24 of the present embodiment is continuous with the cover portion 23, and the support portion 24 supports the cover portion 23 in the continuous place.

As illustrated in Fig. 4B and the like, in a plan view, a part of the support portion 24 of the present embodiment is disposed at a position where the part overlaps the adhesion sheet 2.
In other words, a part of the support portion 24 of the present embodiment is a portion of the holder 21, which overlaps the adhesion sheet 2 in a plan view.
Specifically, the support portion 24 of the present embodiment includes a portion that extends along the adhesion sheet 2 having a C shape in a plan view, and a lower surface of the portion is fixed to the mounting surface 12 of the adhesion sheet 2.
In addition, in a plan view, the support portion 24 of the present embodiment extends to surround the cover portion 23.

The support portion 24 of the present embodiment is fixed to the mounting surface 12, for example, by bonding, fusion, or the like; however, as long as a portion of the adhesion sheet 2 is located in the upward direction A2 above the adhesion surface 11, the position of the portion, at which the support portion 24 is fixed, is not particularly limited.
Therefore, the support portion 24 may be fixed to a layer located in the downward direction A1 below the mounting surface 12.
Meanwhile, with the configuration where the support portion 24 of the device main body 3 is fixed to the mounting surface 12 of the adhesion sheet 2 by bonding, fusion, or the like, the device main body 3 can be easily mounted to the adhesion sheet 2.

### <Release sheet 4>

As described above, the release sheet 4 includes the release layer 4a and the folded layer 4b.
In addition, the folded layer 4b of the release sheet 4 is layered on the release layer 4a at the position of the second portion X2 of the adhesion sheet 2.

The release sheet 4 of the present embodiment has two folds 30, which are linear, at an outer edge of the release layer 4a.
In addition, the release sheet 4 of the present embodiment includes a fold 31 between the folded layers 4b in addition to the folds 30. Hereinafter, for convenience of description, a fold of the two folds 30 which is far from the fold 31 between the folded layers 4b is referred to as a "first fold 30a".
In addition, a fold of the two folds 30 which is close to the fold 31 between the folded layers 4b is referred to as a "second fold 30b".

As illustrated in Fig. 7 and the like, in a plan view, the first fold 30a which is one of the two folds 30 between the release layer 4a and the folded layer 4b of the release sheet 4 passes through the first portion X1 of the adhesion sheet 2 in a plan view.
In other words, in a plan view, at least a part of the first fold 30a overlaps the first portion X1 of the adhesion sheet 2.
The folded layer 4b is moved to cause the release layer 4a of the release sheet 4 to be released from the adhesion surface 11 of the adhesion sheet 2.
For this reason, the release layer 4a is released from the position of the fold 30 with respect to the adhesion surface 11 of the adhesion sheet 2.
With the configuration where the first fold 30a passes through the first portion X1 of the adhesion sheet 2 in a plan view, the position of the first portion X1 of the adhesion sheet 2 can be set as the release start position of the release layer 4a of the release sheet 4.
Since the release layer 4a of the release sheet 4 starts being released from the first portion X1 of the adhesion sheet 2, as compared to when release starts from the second portion X2 of the adhesion sheet 2, the release sheet 4 is more easily released from the adhesion sheet 2.
Namely, the winding and bending of the second portion X2 of the adhesion sheet 2 caused by the release operation of the release sheet 4 can be suppressed.

As illustrated in Fig. 7 and the like, the first fold 30a of the present embodiment passes through the first portion X1 of the adhesion sheet 2 in a plan view, but may be a fold that does not pass through the first portion X1 and the second portion X2 of the adhesion sheet 2 in a plan view.
Even with such a fold, when the second portion X2 does not intervene between the fold and the first portion X1 of the adhesion sheet 2 in a plan view, the same effect as described above can be obtained.
Further, even when the second portion X2 intervenes between the fold and the first portion X1 of the adhesion sheet 2 in a plan view but the length of the second portion X2 is sufficiently short (for example, 2 mm or less), the same effect as described above can be obtained.

As illustrated in Fig. 7 and the like, in a plan view, the second fold 30b which is the other of the two folds 30 between the release layer 4a and the folded layer 4b of the release sheet 4 does not pass through the first portion X1 of the adhesion sheet 2 in a plan view.
However, in a plan view, the second fold 30b does not pass through the first portion X1 and the second portion X2 of the adhesion sheet 2.
In addition, in a plan view, the second portion X2 intervenes between the second fold 30b and the first portion X1 of the adhesion sheet 2, but the length of the second portion X2 is sufficiently short (2 mm or less).
Therefore, the same effect as that in the first fold 30a described above can be obtained even at the position of the second fold 30b.

Here, as illustrated in Fig. 6 and the like, in a plan view, the device main body 3 is interposed between a portion of the first fold 30a, which overlaps the first portion X1 of the adhesion sheet 2, and the extending portion 50 as the designated grip portion of the sheet grip portion which is formed of the folded layer 4b.
With such a configuration, the release sheet 4 is easily released while passing through the first portion X1 of the adhesion sheet 2 on a lower surface of the device main body 3.
Namely, the possibility that the release sheet 4 is released only from the position of the second portion X2 of the adhesion sheet 2 can be reduced.
In other words, when the release sheet 4 is released from a predetermined place on the second portion X2 of the adhesion sheet 2, the possibility that the release sheet 4 is released from a predetermined place on the first portion X1 of the adhesion sheet 2 can be increased.
For this reason, the winding and bending of the second portion X2 of the adhesion sheet 2 caused by the release operation of the release sheet 4 can be suppressed.

Further, as illustrated in Fig. 7 and the like, in a plan view, the holder 21 of the device main body 3, more specifically, the support portion 24 of the holder 21 is interposed between the portion of the first fold 30a, which overlaps the first portion X1 of the adhesion sheet 2, and the extending portion 50 as the designated grip portion of the sheet grip portion which is formed of the folded layer 4b.
As described above, in a plan view, the support portion 24 of the present embodiment extends to surround the cover portion 23.
In addition, in a plan view, a part of the support portion 24 (for example, refer to the position of reference sign "24a" in Fig. 7) of the present embodiment extends from the portion of the first fold 30a, which overlaps the first portion X1 of the adhesion sheet 2, in a direction toward the extending portion 50 as the designated grip portion of the sheet grip portion.
As a consequence, when the extending portion 50 as the designated grip portion is moved to the outside of the device main body 3 in a plan view, the release sheet 4 can be released along the first portion X1 of the adhesion sheet 2 which is located below the support portion 24.
For this reason, the winding and bending of the second portion X2 of the adhesion sheet 2 caused by the release operation of the release sheet 4 can be further suppressed.

In addition, as illustrated in Fig. 7 and the like, in the present embodiment, in a plan view, the extending portion 50 as the designated grip portion of the sheet grip portion is formed at a position different from that of the receiving portion 14 defined by the adhesion sheet 2.
As a consequence, in a state where a portion of the insertion device such as a sheath which is inserted into the living body remains received by the receiving portion 14, the portion extending outside the living body, the extending portion 50 as the designated grip portion of the sheet grip portion can be operated to release the release sheet 4 from the adhesion sheet 2.

Further, as illustrated in Fig. 7 and the like, in a plan view, the release sheet 4 of the present embodiment is not provided in at least a part of the receiving portion 14 defined by the adhesion sheet 2.
Namely, even in a pre-use state before the release sheet 4 is released, the receiving portion 14 defined by the adhesion sheet 2 can receive the insertion device such as a sheath from the outside.

As the release sheet 4, a plastic film made of, for example, polyethylene, polyvinyl chloride, polyester, polypropylene, or the like, paper, a metal film, a cloth, or the like can be used.
In addition, the release sheet 4 may be a single-layer film which is formed of a film of one layer, or may be a laminated film in which a plurality of types of films are laminated.
Further, a release treatment such as a silicon treatment or embossing may be applied to the surface of the release sheet 4, to which the adhesion surface 11 of the adhesion sheet 2 adheres.
The thickness of the release sheet 4 is, for example, from 15 µm to 200 µm, preferably, 40 µm to 100 µm.

In the present embodiment, the tensile modulus of the release sheet 4 is smaller than the tensile modulus of the adhesion sheet 2.
Namely, both the adhesion sheet 2 and the release sheet 4 are flexible, and in the present embodiment, the adhesion sheet 2 has a higher flexibility and is more easily bent than the release sheet 4.
For this reason, when the release sheet 4 is tried to be released from the second portion X2 of the adhesion sheet 2, the second portion X2 of the adhesion sheet 2 is easily bent and deformed according to the release sheet 4.
Meanwhile, the tensile modulus of the release sheet 4 may be the tensile modulus of the adhesion sheet 2 or more.

### <Compression method performed using adhesion device 1>

Next, a method of compressing a biological surface, which is performed using the adhesion device 1, will be described.

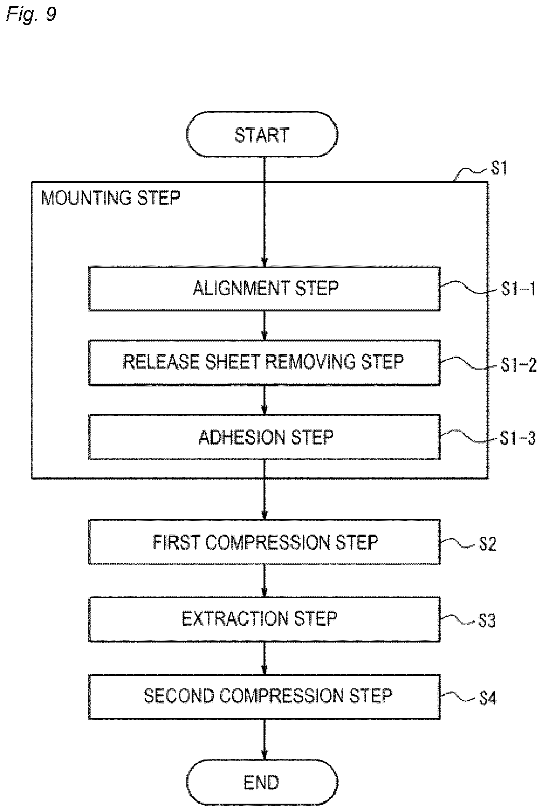

Fig. 9 is a flowchart illustrating one example of the method of compressing a biological surface.
The compression method illustrated in Fig. 9 includes a mounting step S1, a first compression step S2, an extraction step S3, and a second compression step S4.
Figs. 10A to 10E are views illustrating an outline of the mounting step S1.

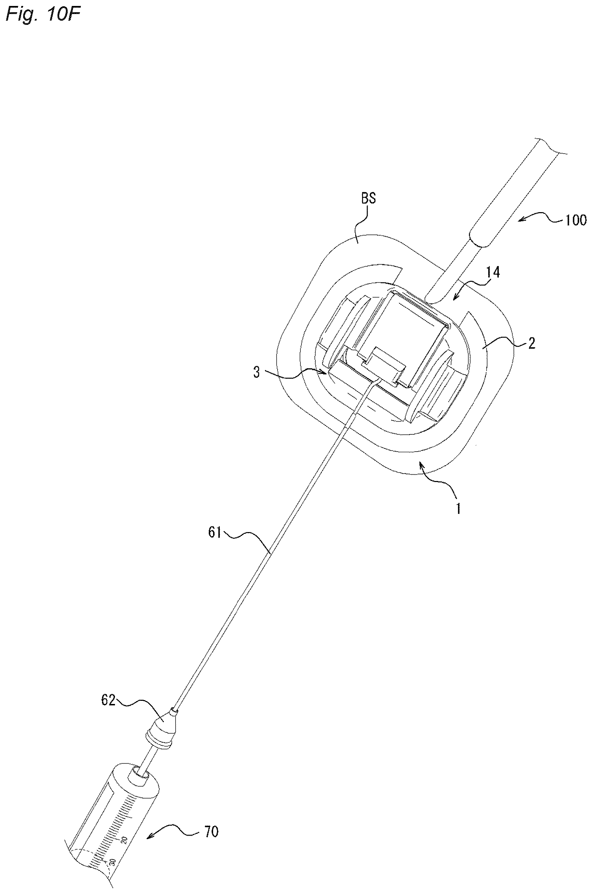

Fig. 10F is a view illustrating an outline of the first compression step S2.

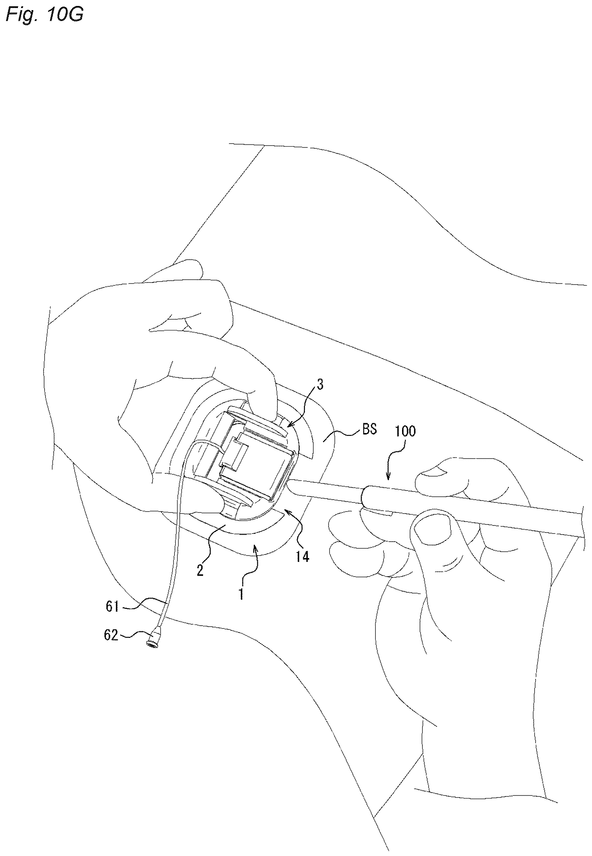

Fig. 10G is a view illustrating an outline of the extraction step S3.

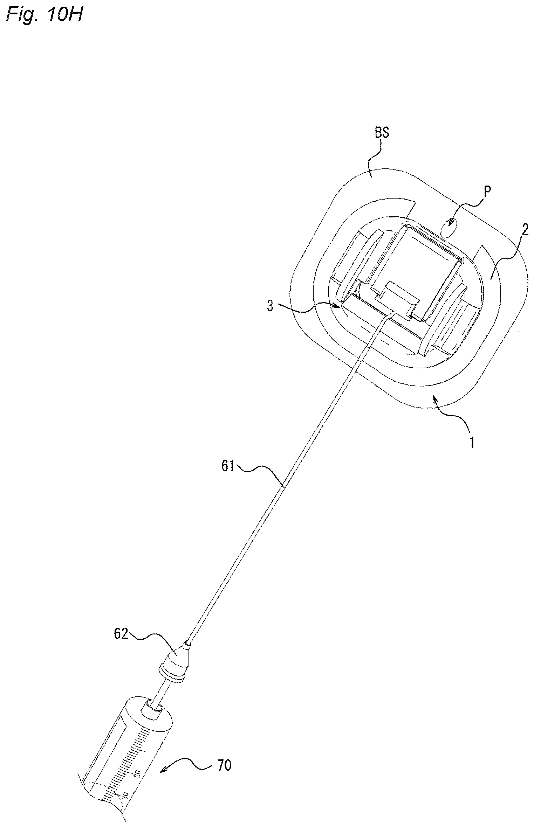

Fig. 10H is a view illustrating an outline of the second compression step S4.

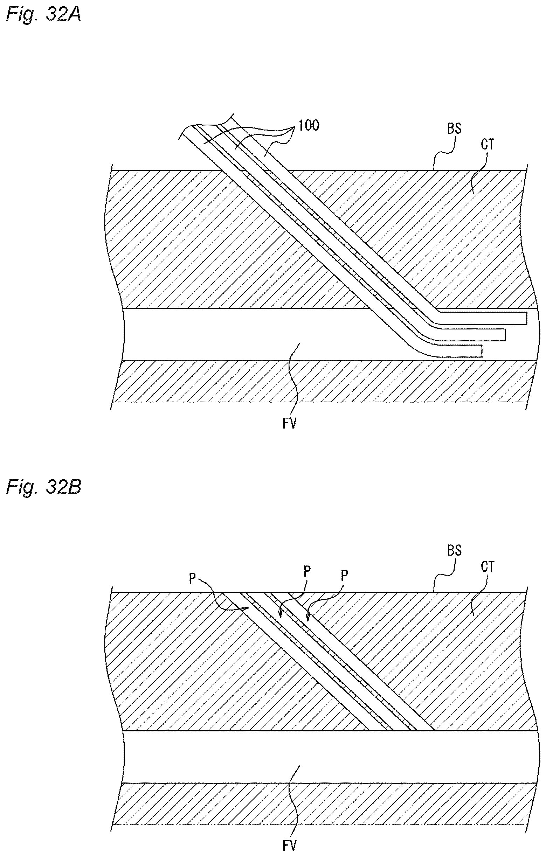

The compression method illustrated in Figs. 9 and 10A to 10H is a compression method by which the biological surface BS is compressed to narrow or obstruct a perforation, which is formed by extraction of a sheath as an insertion device 100 in the state of being inserted into, for example, a vein such as a femoral vein from the biological surface BS through a connective tissue, without obstructing the vein.
Accordingly, hemostasis can be performed after the sheath as the insertion device 100 is extracted.
First, the perforation which is formed after the insertion device 100 is extracted will be described with reference to Figs. 32A and 32B.
Fig. 32A illustrates a state where the sheath as the insertion device 100 is inserted into a femoral vein FV from the biological surface BS through a connective tissue CT.
Fig. 32A illustrates three sheaths as the insertion device 100; however, the number of the sheaths may be two or less or may be four or more.
Fig. 32B illustrates a state of the sheath as the insertion device 100 after extracted from the state illustrated in Fig. 32A.
As illustrated in Fig. 32B, when the sheaths as the insertion device 100 are extracted, perforations P are formed between the biological surface BS and the femoral vein FV.
In the compression method illustrated in Figs. 9 and 10A to 10H, the perforations P can be narrowed or obstructed without obstructing the femoral vein FV.
Hereinafter, steps S1 to S4 will be described in detail with reference to Figs. 10A to 10H.

Fig. 10A illustrates a state where the sheath as the insertion device 100 is inserted into the femoral vein FV from the biological surface BS (refer to Figs. 32A and 32B).
First, in this state, the adhesion device 1 is mounted on the biological surface BS.

As illustrated in Fig. 9, the mounting step S1 includes an alignment step S1-1 of aligning the mounting position of the adhesion device 1 on the biological surface BS, a release sheet removing step S1-2 of releasing the release sheet 4 from the adhesion surface 11 of the adhesion sheet 2, and an adhesion step S1-3 of causing the adhesion surface 11 of the adhesion sheet 2 to adhere to the biological surface BS.

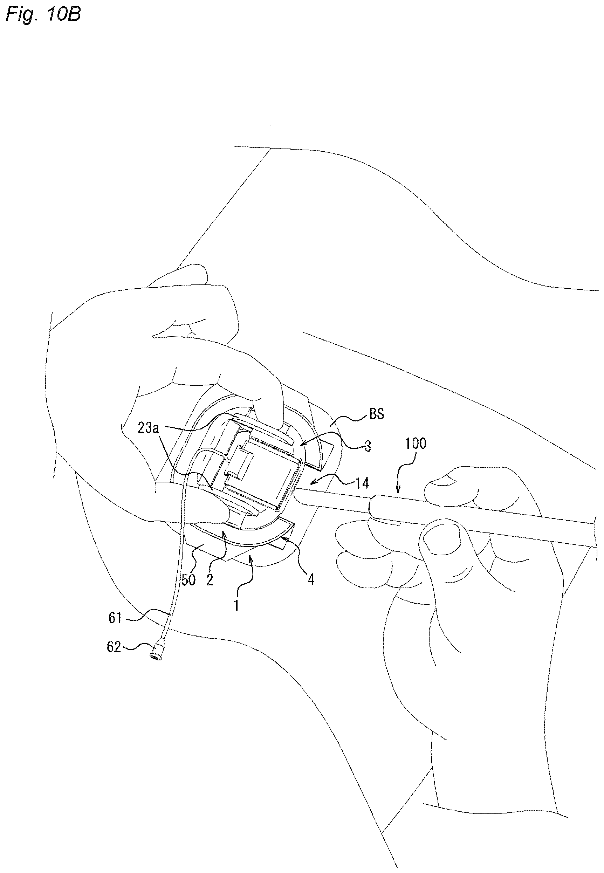

Figs. 10A and 10B are views illustrating an outline of the alignment step S1-1 of the mounting step S1.
As illustrated in Fig. 10B, in the alignment step S1-1, the adhesion position of the adhesion device 1 is aligned such that a portion of the sheath as the insertion device 100 is received by the receiving portion 14, the portion extending outside the living body.

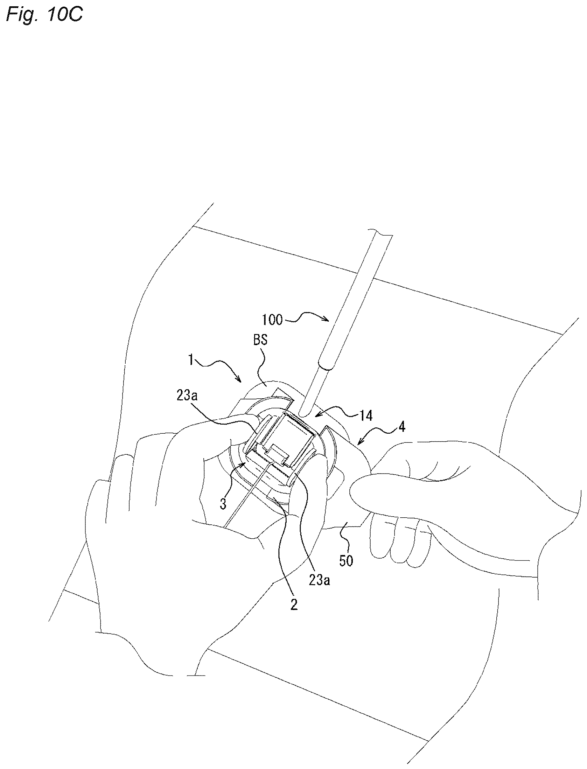

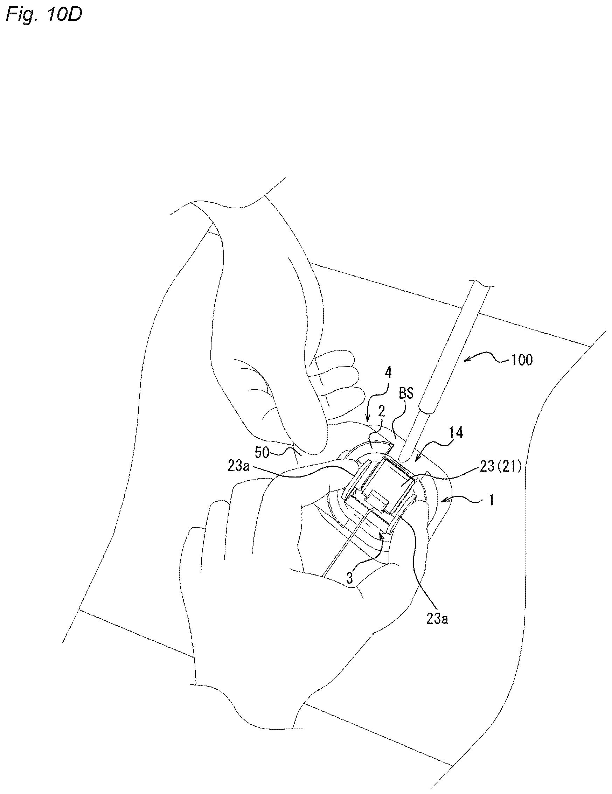

Figs. 10C and 10D are views illustrating an outline of the release sheet removing step S1-2 of the mounting step S1.
As illustrated in Figs. 10C and 10D, in the release sheet removing step S1-2, an operator such as a health care worker releases the release sheet 4 from the adhesion sheet 2 while holding the adhesion device 1 in a place where the alignment is made in the alignment step S1-1.
As illustrated in Figs. 10C and 10D, in a state where the device main body 3 remains held with one hand of the operator, the release sheet 4 is released with the other hand of the operator.
As a consequence, the adhesion device 1 is easily held in the place where the alignment is made in the alignment step S1-1.
For this reason, it is preferable that the device main body 3 is provided with a grip portion which is easy for the operator to grip. As described above, in the present embodiment, the grip portion is formed of the two plate portions 23a provided in the cover portion 23 of the holder 21 of the device main body 3.

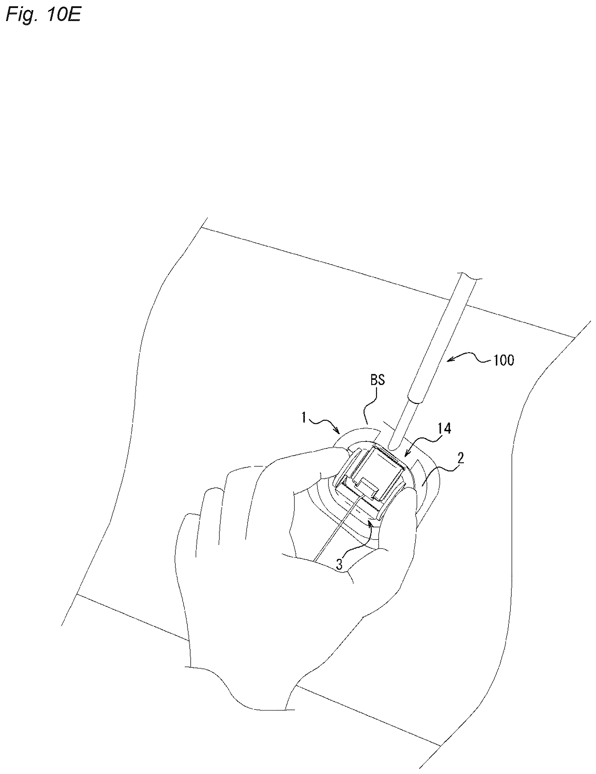

Fig. 10E is a view illustrating an outline of the adhesion step S1-3 of the mounting step S1.
As illustrated in Fig. 10E, in the adhesion step S1-3, the adhesion surface 11 of the adhesion sheet 2, which is exposed in the release sheet removing step S1-2, adheres to the biological surface BS.
The place where adhesion is performed in the adhesion step S1-3 is a place on the biological surface BS, in which the alignment is made in the alignment step S1-1.
In such a manner, the adhesion device 1 can be mounted in a predetermined place on the biological surface BS.

Next, as illustrated in Fig. 10F, a syringe 70 as a fluid supply device is connected to the connection section 62 of the tube 61.
Air is supplied to the inflator 55 (refer to Fig. 8B) as the compression member 22 of the adhesion device 1 through the tube 61 to inflate the inflator 55.
As a consequence, before the sheath as the insertion device 100 is extracted from the biological surface BS, the vicinity of a wound on the biological surface BS can be compressed in advance.
In other words, the compression of the biological surface BS starts in a state where the sheath as the insertion device 100 is inserted into the femoral vein FV as a vein from the biological surface BS through the connective tissue CT (refer to Figs. 32A and 32B).
As described above, since compression is performed before the insertion device 100 is extracted from the biological surface BS, immediately after the sheath as the insertion device 100 is extracted, the biological surface BS can be compressed such that the perforations P (refer to Fig. 32B) extending from the biological surface BS to the femoral vein FV (refer to Figs. 32A and 32B) are narrowed or obstructed.

Next, as illustrated in Fig. 10G, the sheath as the insertion device 100 is extracted from the biological surface BS.
More specifically, the sheath as the insertion device 100 of the present embodiment is extracted from the living body through the receiving portion 14 of the adhesion sheet 2.
The perforations P illustrated in Fig. 32B are formed by extraction of the sheath.
If the biological surface BS is not compressed at all in this state, bleeding occurs from the femoral vein FV to the outside of the living body through the perforations P and the wound on the biological surface BS.
However, in the compression method illustrated here, as illustrated in Fig. 10F, before the sheath as the insertion device 100 is extracted from the biological surface BS, the biological surface BS is compressed in advance.
For this reason, immediately after the sheath is extracted, the biological surface BS can be compressed such that the perforations P (refer to Fig. 32B) are narrowed or obstructed, and the amount of bleeding immediately after extraction of the sheath can be suppressed.

Next, as illustrated in Fig. 10H, the syringe 70 as a fluid supply device is connected again to the connection section 62 of the tube 61. Air is supplied again to the inflator 55 (refer to Fig. 8B) as the compression member 22 of the adhesion device 1 through the tube 61 to apply pressure, or is removed to reduce pressure. In other words, the compression force of the biological surface BS after extraction of the sheath as the insertion device 100 is adjusted. Accordingly, the compression force of the biological surface BS is adjusted to further narrow or obstruct the perforations P (refer to Fig. 32B) without obstructing the femoral vein FV (refer to Figs. 32A and 32B), so that the amount of bleeding can be greatly reduced or bleeding can be stopped.

More specifically, when bleeding is confirmed after the sheath is extracted, the compression force is slowly increased to apply pressure until hemostasis is achieved. On the other hand, when hemostasis is confirmed after the sheath is extracted, the compression force is slowly decreased to reduce pressure until bleeding is confirmed. Then, after bleeding is confirmed, the compression force is slowly increased to apply pressure until hemostasis is achieved. As a consequence, the obstruction of the femoral vein FV (refer to Figs. 32A and 32B) by overpressurization can be prevented.

The compression state is maintained for several hours (for example, two to six hours) as it is, so that hemostasis can be completed. After hemostasis is completed, the adhesion surface 11 of the adhesion sheet 2 is released from the biological surface BS, so that the adhesion device 1 is removed from the biological surface BS.

In the compression method illustrated here, the perforations P (refer to Fig. 32B) are narrowed or obstructed without obstructing the femoral vein FV (refer to Figs. 32A and 32B). In the case of hemostasis of a vein, hemostasis can be performed by narrowing or obstructing the perforations P (refer to Fig. 32B). Meanwhile, for example, in the case of hemostasis of a femoral artery, even when only perforations are obstructed, blood leaks and spreads in the connective tissue CT (refer to Figs. 32A and 32B), so that hemostasis cannot be achieved. In the case of hemostasis of the femoral artery, a large-scale measure such as a method of applying strong compression to the extent that the artery itself is narrowed or obstructed or a method of closing a hole in an artery wall is required.

Therefore, in the above-described compression method, it is preferable that the biological surface BS is compressed to a position where the compression depth from the biological surface BS is from 5 mm to 20 mm. When the compression depth is set in the above range, a compression state where the perforations P (refer to Fig. 32B) are narrowed or obstructed without obstructing the vein is easily realized. The compression depth is more preferably set in a range of 5 mm to 15 mm, further preferably, in a range of 8 mm to 12 mm. Further, as illustrated in Fig. 8B, in the present embodiment, the inflator 55 as the compression member 22 includes the second inflator 60b as the pressing portion 22b. For this reason, according to the compression member 22 of the present embodiment, the compression force in a direction orthogonal to an extending direction of the perforations P (refer to Fig. 32B) can be increased. Accordingly, the compression state where the perforations P (refer to Fig. 32B) are narrowed or obstructed without obstructing the vein is more easily realized.

Further, in the above-described compression method, it is preferable that the biological surface BS is compressed at 25 g/cm² to 400 g/cm² from the biological surface BS. The compression pressure is a pressure after the sheath as the insertion device 100 is extracted, and does not mean the above-described compression force before extraction of the sheath. When the compression pressure is set in the above range, the compression state where the perforations P (refer to Fig. 32B) are narrowed or obstructed without obstructing the vein is easily realized. The compression pressure is more preferably set in a range of 50 g/cm² to 400 g/cm², further preferably, in a range of 100 g/cm² to 300 g/cm².

According to the compression method illustrated in Figs. 9 and 10A to 10H, hemostasis can be performed by narrowing or obstructing the perforations P (refer to Fig. 32B) without obstructing the vein such as the femoral vein FV. Particularly, the above-described compression method is realized by the adhesion device 1, so that compression by the hand of a health care worker, the use of a large-scale hemostat device, or the like is not required, and hemostasis can be performed by a simple method. Further, as illustrated in Fig. 32B, even when a plurality of the perforations P are collectively formed, the plurality of perforations P can be collectively narrowed or obstructed.

### [Second embodiment]

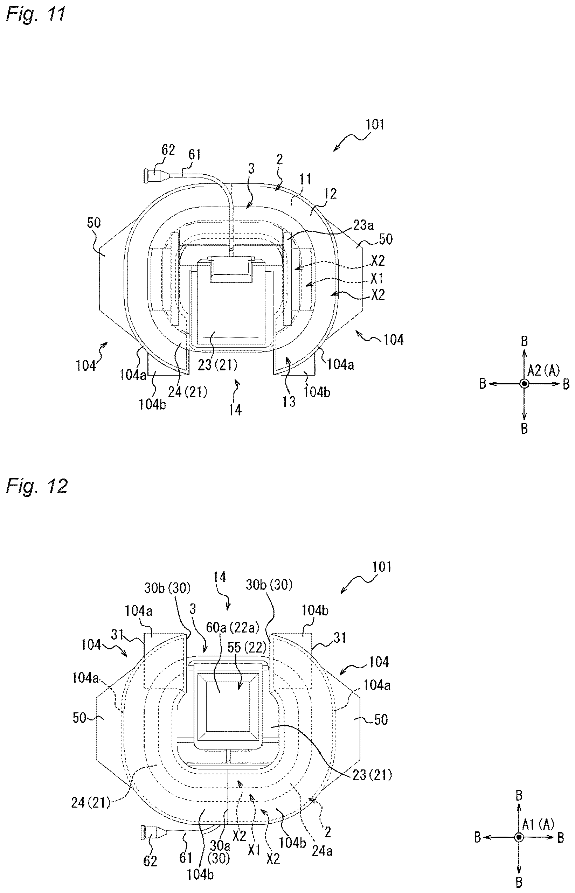

Next, an adhesion device 101 in a second embodiment will be described with reference to Figs. 11 to 15.
Fig. 11 is a top view of the adhesion device 101 in the present embodiment. Fig. 12 is a bottom view of the adhesion device 101.

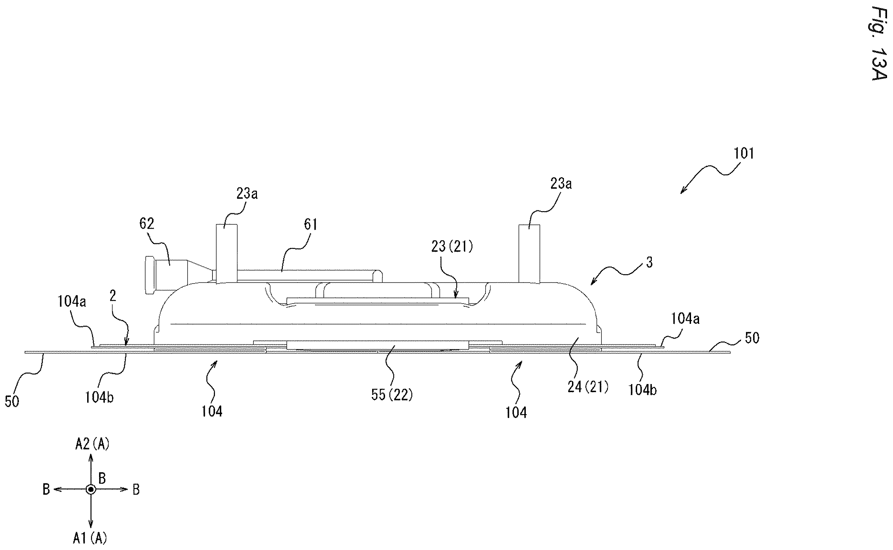

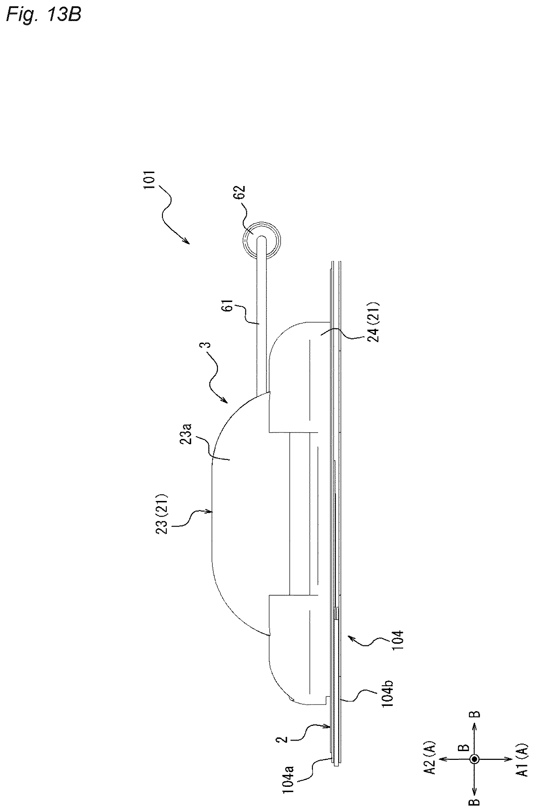

Figs. 13A and 13B are side views of the adhesion device 101. Figs. 13A and 13B are side views seen from different viewpoints. Fig. 14 is a view illustrating a release sheet 104 of the adhesion device 101. Fig. 14 illustrates a method of forming the release sheet 104.

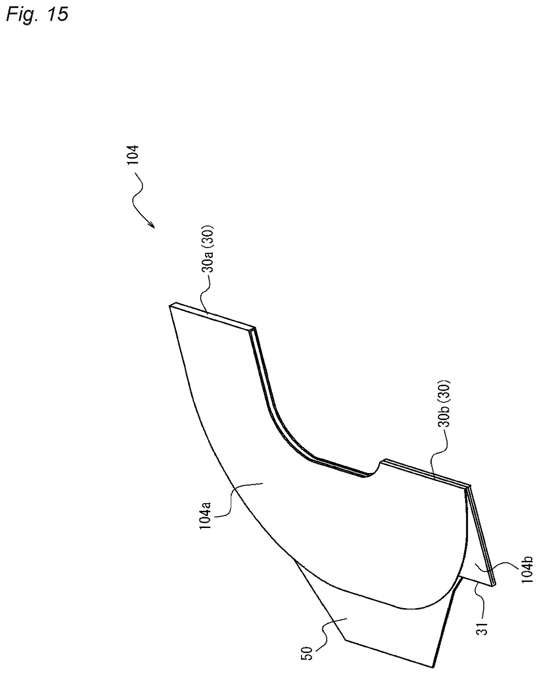

Fig. 15 is a perspective view of the release sheet 104 of the adhesion device 101.

The adhesion device 101 includes the adhesion sheet 2, the device main body 3, and the release sheet 104. Since the adhesion sheet 2 and the device main body 3 of the adhesion device 101 are the same as those of the adhesion device 1 of the first embodiment, the description thereof will be omitted here. In addition, the release sheet 104 is different in folding method from the release sheet 4 (refer to Fig. 1 and the like) of the first embodiment, and other configurations are the same. Therefore, here, the points of difference of the release sheet 104 of the present embodiment over the release sheet 4 described above will be mainly described, and the

As illustrated in Figs. 14 and 15, the release sheet 104 includes a release layer 104a and a folded layer 104b. The configuration of the release layer 104a of the release sheet 104 is the same as that of the release layer 4a of the release sheet 4 described above. Meanwhile, the folded layer 104b of the release sheet 104 is different from the folded layer 4b of the release sheet 4 described above. As illustrated in Fig. 15, a part of the folded layer 104b of the release sheet 104 may include three layers that are folded in a zigzag shape.

### [Third embodiment]

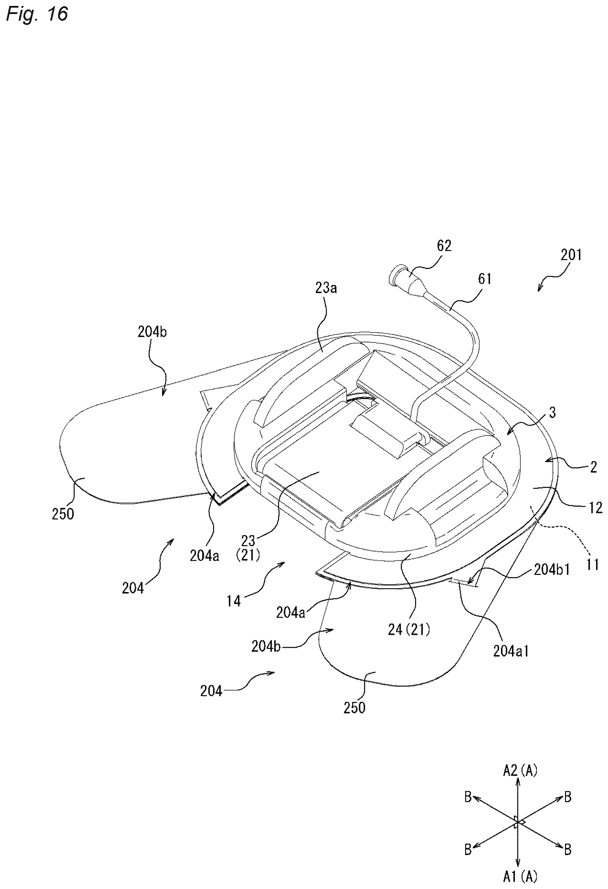

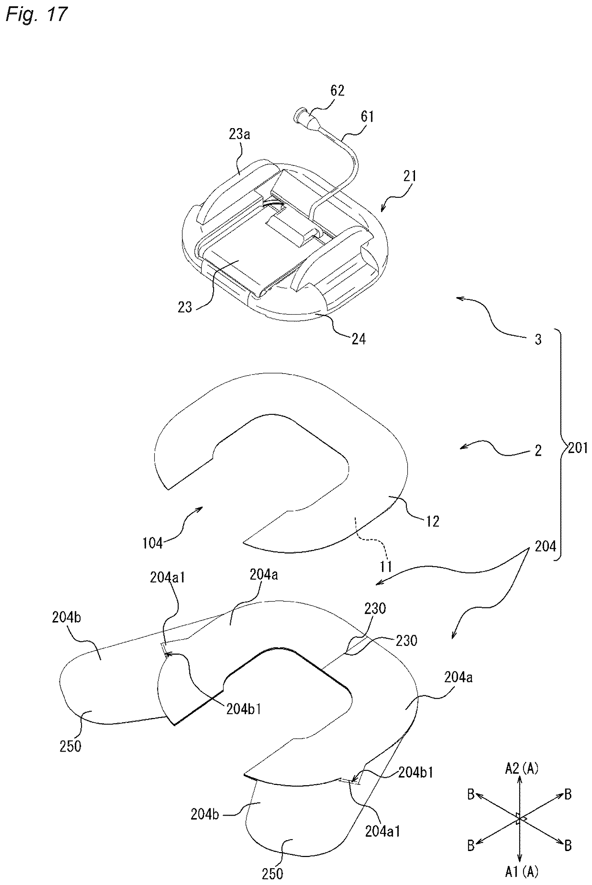

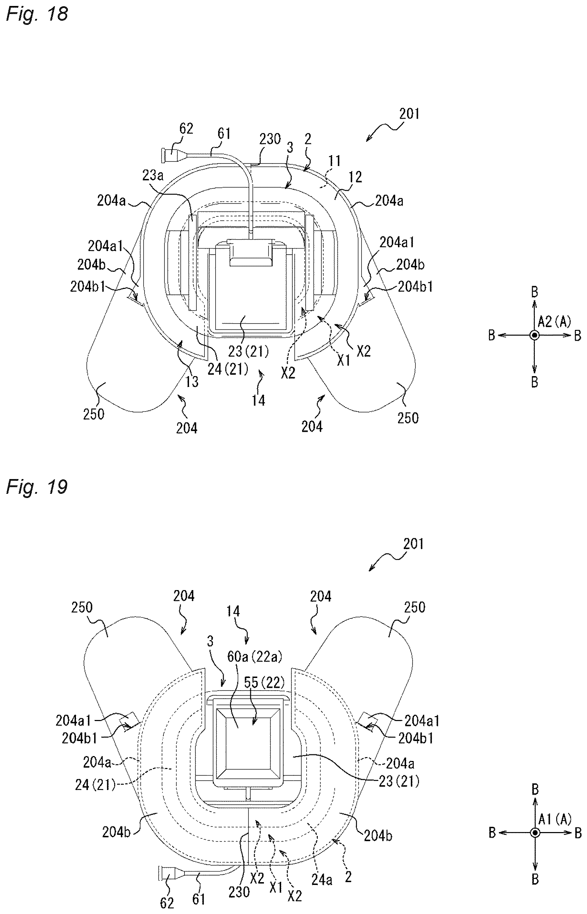

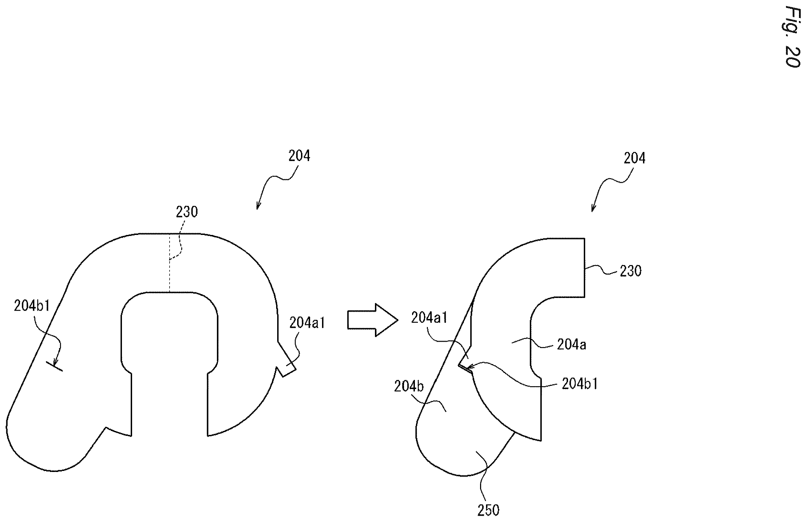

Next, an adhesion device 201 in a third embodiment will be described with reference to Figs. 16 to 20. Fig. 16 is a perspective view of the adhesion device 201. Fig. 17 is an exploded perspective view of the adhesion device 201. Fig. 18 is a top view of the adhesion device 201. Fig. 19 is a bottom view of the adhesion device 201. Fig. 20 is a view illustrating a release sheet 204 of the adhesion device 201. Fig. 20 illustrates a method of forming the release sheet 204.

The adhesion device 201 includes the adhesion sheet 2, the device main body 3, and the release sheet 204. Since the adhesion sheet 2 and the device main body 3 of the adhesion device 201 are the same as those of the adhesion device 1 of the first embodiment, the description thereof will be omitted here.

The release sheet 204 releasably adheres to the adhesion surface 11 of the adhesion sheet 2. When the adhesion device 201 adheres to the biological surface, the release sheet 204 is released from the adhesion surface 11. Accordingly, the adhesion surface 11 of the adhesion sheet 2 is exposed to the outside, and the adhesion device 201 is in the state of being able to adhere to the biological surface.

As illustrated in Figs. 16 to 20, the release sheet 204 includes a release layer 204a that adheres to the adhesion surface 11 of the adhesion sheet 2, and a folded layer 204b which is formed of at least one layer (one layer in the present embodiment) and is folded to a side, which is opposite to the adhesion surface 11 with respect to the release layer 204a, to be layered.

As illustrated in Fig. 16, the folded layer 204b of the release sheet 204 is layered on the release layer 204a of the release sheet 204 at least at the position of the second portion X2 of the adhesion sheet 2. Since the release sheet 204 has such a configuration, similar to the release sheet 4 of the first embodiment, the release sheet 204 is easily released from the second portion X2 of the adhesion sheet 2 (refer to Fig. 18 and the like).

As illustrated in Fig. 17 and the like, in the present embodiment, the entire region of the adhesion surface 11 of the adhesion sheet 2 is covered with two release sheets 204 which are symmetrical in shape. Fig. 20 illustrates only one release sheet 204 of the two release sheets 204 which are symmetrical in shape. Since the two release sheets 204 are symmetrical in shape, hereinafter, only the one release sheet 204 will be described as an example. As illustrated in Fig. 20, the release sheet 204 of the present embodiment is folded once from an original form of a single layer (left figure in Fig. 20) to be shaped into a completed form of two layers (right figure in Fig. 20). In such a manner, the release sheet 204 including the release layer 204a which is formed of one layer and the folded layer 204b which is formed of one layer is formed.

As illustrated in Fig. 20, the original form (left figure in Fig. 20) of a single layer in the release sheet 204 of the present embodiment has a substantially C-shaped or a substantially U-shaped outer shape in a plan view seen in the thickness direction. In other words, the original form (left figure in Fig. 20) of the release sheet 204 of the present embodiment is not formed in an endless shape in a plan view seen in the thickness direction, and a gap is provided in a part of the original form. In addition, the release sheet 204 of the present embodiment is folded such that a fold 230 in the completed form (right figure in Fig. 20) is formed in one place. In other words, a virtual straight line obtained by extending only one fold 230 which is linear passes through the above-described gap of the release sheet 204 in the original form (left figure in Fig. 20) unfolded from the completed form (right figure in Fig. 20).

As described above, the release sheet 204 of the present embodiment has only one fold 230 between the release layer 204a and the folded layer 204b. In addition, the fold 230 is disposed to be biased toward one end when the release sheet 204 is in the completed form (right figure in Fig. 20). For this reason, a latch section which latches one of the release layer 204a and the folded layer 204b to the other is provided at the position of the other end, which is opposite to the one end where the fold 230 is provided, when the release sheet 204 is in the completed form (right figure in Fig. 20). Accordingly, the other end which is far from the fold 230 of the folded layer 204b can be suppressed from hanging down from the release layer 204a. The latch section of the present embodiment is formed of a projection portion 204a1 of the release layer 204a, and a slit 204b1 of the folded layer 204b. The projection portion 204a1 is formed in a part of an outer edge of the release layer 204a. The projection portion 204a1 can be inserted into the slit 204b1 when the release sheet 204 is in the completed form (right figure in Fig. 20). The latch section is not limited to the above-described configuration illustrated in the present embodiment. For example, the projection portion may be provided in the folded layer, and the slit may be provided in the release layer. It is preferable that since a latch state can be easily released, the latch section formed of the projection portion and the slit described above is employed.

As illustrated in Fig. 19 and the like, in a plan view, the fold 230 between the release layer 204a and the folded layer 204b of the release sheet 204 passes through the first portion X1 of the adhesion sheet 2. As a consequence, similar to the release sheet 4 (refer to Fig. 1 and the like) described above, the position of the first portion X1 of the adhesion sheet 2 can be set as a release start position of the release layer 204a of the release sheet 204. Accordingly, similar to the release sheet 4 (refer to Fig. 1 and the like) described above, the winding and bending of the second portion X2 of the adhesion sheet 2 caused by the release operation of the release sheet 204 can be suppressed.

Here, as illustrated in Fig. 18 and the like, in a plan view, the device main body 3 is interposed between a portion of the fold 230, which overlaps the first portion X1 of the adhesion sheet 2, and an extending portion 250 as a designated grip portion of a sheet grip portion which is formed of the folded layer 204b. With such a configuration, similar to the release sheet 4 (refer to Fig. 1 and the like) described above, the release sheet 204 is easily released while passing through the first portion X1 of the adhesion sheet 2 on the lower surface of the device main body 3. Namely, similar to the release sheet 4 (refer to Fig. 1 and the like) described above, the winding and bending of the second portion X2 of the adhesion sheet 2 caused by the release operation of the release sheet 204 can be suppressed.

Further, as illustrated in Fig. 18 and the like, in a plan view, the holder 21 of the device main body 3, more specifically, the support portion 24 of the holder 21 is interposed between the portion of the fold 230, which overlaps the first portion X1 of the adhesion sheet 2, and the extending portion 250 as the designated grip portion of the sheet grip portion which is formed of the folded layer 204b. As illustrated in Fig. 19 and the like, in a plan view, the support portion 24 of the present embodiment extends to surround the cover portion 23. In addition, in a plan view, a part of the support portion 24 (for example, refer to the position of reference sign "24a" in Fig. 19) of the present embodiment extends from the portion of the fold 230, which overlaps the first portion X1 of the adhesion sheet 2, in a direction toward the extending portion 250 as the designated grip portion of the sheet grip portion. As a consequence, when the extending portion 250 as the designated grip portion is moved to the outside of the device main body 3 in a plan view, the release sheet 204 can be released along the first portion X1 of the adhesion sheet 2 which is located below the support portion 24. For this reason, similar to the release sheet 4 (refer to Fig. 1 and the like) described above, the winding and bending of the second portion X2 of the adhesion sheet 2 caused by the release operation of the release sheet 204 can be further suppressed.

In addition, as illustrated in Fig. 18 and the like, in the present embodiment, in a plan view, the extending portion 250 as the designated grip portion of the sheet grip portion is formed at a position different from that of the receiving portion 14 defined by the adhesion sheet 2. As a consequence, in a state where a portion of the insertion device such as a sheath which is inserted into the living body remains received by the receiving portion 14, the portion extending outside the living body, the extending portion 250 as the designated grip portion of the sheet grip portion can be operated to release the release sheet 204 from the adhesion sheet 2.

A mark which guides an operator such as a health care worker through a predetermined operation may be formed in the extending portion 250 as the designated grip portion of the sheet grip portion. Examples of such a mark include various marks which guide that the extending portion 250 is to be gripped to release the release sheet 204, various marks such as arrows which guide a direction in which the extending portion 250 is moved when the release sheet 204 is released, and the like.

Further, as illustrated in Fig. 18 and the like, in a plan view, the release sheet 204 of the present embodiment is not provided in at least a part of the receiving portion 14 defined by the adhesion sheet 2. Namely, even in a pre-use state before the release sheet 204 is released, the receiving portion 14 defined by the adhesion sheet 2 can receive the insertion device 100 such as a sheath (refer to Fig. 10A and the like) from the outside.

### [Fourth embodiment]

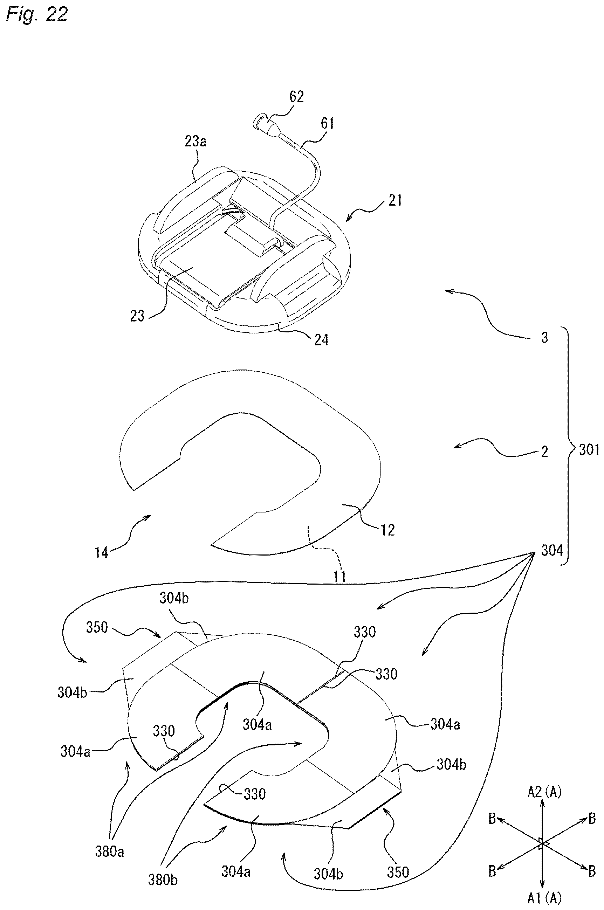

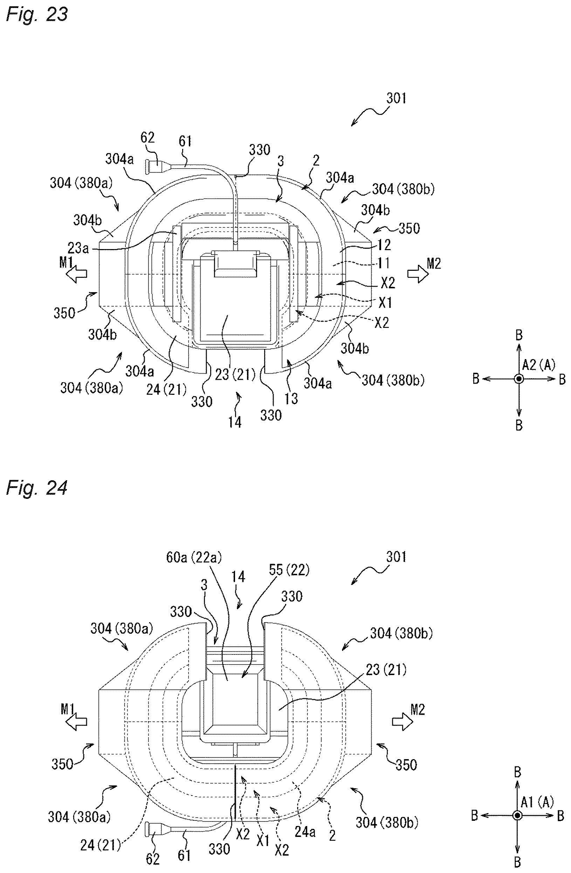

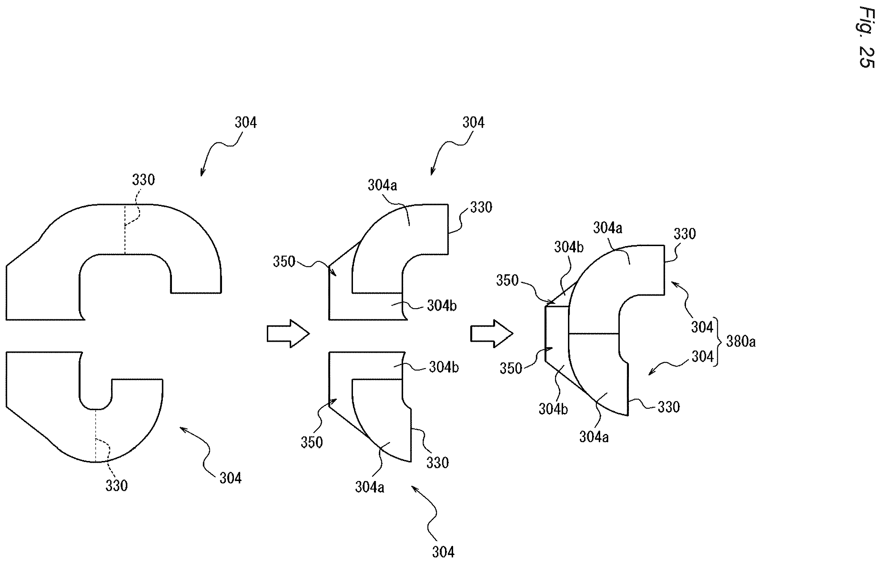

Next, an adhesion device 301 in a fourth embodiment will be described with reference to Figs. 21 to 25. Fig. 21 is a perspective view of the adhesion device 301. Fig. 22 is an exploded perspective view of the adhesion device 301. Fig. 23 is a top view of the adhesion device 301. Fig. 24 is a bottom view of the adhesion device 301. Fig. 25 is a view illustrating a release sheet 304 of the adhesion device 301. Fig. 25 illustrates a method of forming the release sheet 304.

The adhesion device 301 includes the adhesion sheet 2, the device main body 3, and the release sheet 304. Since the adhesion sheet 2 and the device main body 3 of the adhesion device 301 are the same as those of the adhesion device 1 of the first embodiment, the description thereof will be omitted here.

The release sheet 304 releasably adheres to the adhesion surface 11 of the adhesion sheet 2. When the adhesion device 301 adheres to the biological surface, the release sheet 304 is released from the adhesion surface 11. Accordingly, the adhesion surface 11 of the adhesion sheet 2 is exposed to the outside, and the adhesion device 301 is in the state of being able to adhere to the biological surface.

As illustrated in Figs. 21 to 25, the release sheet 304 includes a release layer 304a that adheres to the adhesion surface 11 of the adhesion sheet 2, and a folded layer 304b which is formed of at least one layer (one layer in the present embodiment) and is folded to a side, which is opposite to the adhesion surface 11 with respect to the release layer 304a, to be layered.

As illustrated in Fig. 21, the folded layer 304b of the release sheet 304 is layered on the release layer 304a of the release sheet 304 at least at the position of the second portion X2 of the adhesion sheet 2. Since the release sheet 304 has such a configuration, similar to the release sheet 4 of the first embodiment, the release sheet 304 is easily released from the second portion X2 of the adhesion sheet 2 (refer to Fig. 23 and the like).

As illustrated in Fig. 22 and the like, in the present embodiment, the entire region of the adhesion surface 11 of the adhesion sheet 2 is covered with two sets of pairs of release sheet groups 380a and 380b which are symmetrical in shape. Fig. 25 illustrates only one release sheet group 380a of the two sets of release sheet groups 380a and 380b which are symmetrical in shape. Since the two sets of release sheet groups 380a and 380b are symmetrical in shape, hereinafter, only one release sheet group 380a will be described as an example.

The release sheet group 380a includes two release sheets 304. As illustrated in Fig. 25, each of the two release sheets 304 of the present embodiment is folded once from an original form of a single layer (left figure in Fig. 25) to be shaped into a completed form of two layers (middle figure in Fig. 25). In such a manner, each of the two release sheets 304 including the release layer 304a which is formed of one layer and the folded layer 304b which is formed of one layer is formed.

Next, parts of the two release sheets 304 overlap each other in the thickness direction, so that the release sheet group 380a (right figure in Fig. 25) is formed. Specifically, in the present embodiment, the release layers 304a of the two release sheets 304 do not overlap each other in the thickness direction. The folded layer 304b of one release sheet 304 overlaps the release layer 304a and the folded layer 304b of the other release sheet 304 in the thickness direction. In addition, the folded layer 304b of the other release sheet 304 overlaps the release layer 304a and the folded layer 304b of the one release sheet 304 in the thickness direction. In such a manner, the release sheet group 380a (right figure in Fig. 25) is formed.

As illustrated in Fig. 25, each of the release sheets 304 of the release sheet group 380a of the present embodiment has only one fold 330 between the release layer 304a and the folded layer 304b.

As illustrated in Fig. 24 and the like, in a plan view, the fold 330 between the release layer 304a and the folded layer 304b of the one release sheet 304 (the release sheet 304 on the upper side in Fig. 25) of the release sheet group 380a passes through the first portion X1 of the adhesion sheet 2. As a consequence, similar to the release sheet 4 (refer to Fig. 1 and the like) described above, the position of the first portion X1 of the adhesion sheet 2 can be set as a release start position of the release layer 304a of the one release sheet 304 (the release sheet 304 on the upper side in Fig. 25) of the release sheet group 380a. Accordingly, similar to the release sheet 4 (refer to Fig. 1 and the like) described above, the winding and bending of the second portion X2 of the adhesion sheet 2 caused by the release operation of the release sheet 304 can be suppressed.

Here, in the one release sheet 304 (the release sheet 304 on the upper side in Fig. 25) of the release sheet group 380a, attention is paid to the folded layer 304b and the fold 330. As illustrated in Fig. 23 and the like, in a plan view, the device main body 3 is interposed between a portion of the fold 330, which overlaps the first portion X1 of the adhesion sheet 2, and an extending portion 350 as a designated grip portion of a sheet grip portion which is formed of the folded layer 304b. With such a configuration, similar to the release sheet 4 (refer to Fig. 1 and the like) described above, the one release sheet 304 (the release sheet 304 on the upper side in Fig. 25) of the release sheet group 380a is easily released while passing through the first portion X1 of the adhesion sheet 2 on the lower surface of the device main body 3. Namely, similar to the release sheet 4 (refer to Fig. 1 and the like) described above, the winding and bending of the second portion X2 of the adhesion sheet 2 caused by the release operation of the one release sheet 304 (the release sheet 304 on the upper side in Fig. 25) of the release sheet group 380a can be suppressed.

On the other hand, as illustrated in Fig. 24 and the like, in a plan view, the fold 330 between the release layer 304a and the folded layer 304b of the other release sheet 304 (the release sheet 304 on the lower side in Fig. 25) of the release sheet group 380a does not pass through the first portion X1 of the adhesion sheet 2. However, in a plan view, the fold 330 of the other release sheet 304 (the release sheet 304 on the lower side in Fig. 25) does not pass through both the first portion X1 and the second portion X2 of the adhesion sheet 2. In addition, in a plan view, the second portion X2 intervenes between the fold 330 and the first portion X1 of the adhesion sheet 2, but the length of the second portion X2 is sufficiently short (2 mm or less). Therefore, the same effect as that in the fold 330 of the one release sheet 304 (the release sheet 304 on the upper side in Fig. 25) can be obtained even at the position of the fold 330 of the other release sheet 304 (the release sheet 304 on the lower side in Fig. 25) of the release sheet group 380a.

Here, in the other release sheet 304 (the release sheet 304 on the lower side in Fig. 25) of the release sheet group 380a, attention is paid to the folded layer 304b and the fold 330. As illustrated in Fig. 23 and the like, in a plan view, the device main body 3 is interposed between the fold 330 and the extending portion 350 as the designated grip portion of the sheet grip portion which is formed of the folded layer 304b. With such a configuration, similar to the release sheet 4 (refer to Fig. 1 and the like) described above, the other release sheet 304 (the release sheet 304 on the lower side in Fig. 25) of the release sheet group 380a is easily released while passing through the first portion X1 of the adhesion sheet 2 on the lower surface of the device main body 3. Namely, similar to the release sheet 4 (refer to Fig. 1 and the like) described above, the winding and bending of the second portion X2 of the adhesion sheet 2 caused by the release operation of the other release sheet 304 (the release sheet 304 on the lower side in Fig. 25) of the release sheet group 380a can be suppressed.

As described above, according to two release sheets 304 of the release sheet group 380a, the winding and bending of the second portion X2 of the adhesion sheet 2 caused by a release operation can be suppressed. In addition, the same effect can be also obtained for the two release sheets 304 of the release sheet group 380b, which are symmetrical in shape.

Hereinafter, items common to four release sheets 304 of the release sheet groups 380a and 380b will be described. For this reason, unless otherwise specified, the four release sheets 304 will be described without distinction.

Attention is paid to each of the release sheets 304. As illustrated in Fig. 23 and the like, in a plan view, the holder 21 of the device main body 3, more specifically, the support portion 24 of the holder 21 is interposed between the fold 330 and the extending portion 350 as the designated grip portion of the sheet grip portion which is formed of the folded layer 304b. The support portion 24 of the present embodiment extends to surround the cover portion 23 in a plan view. In addition, as illustrated in Fig. 24 and the like, in a plan view, a part of the support portion 24 (for example, refer to the position of reference sign "24a" in Fig. 24) of the present embodiment extends from the fold 330 in a direction toward the extending portion 350 as the designated grip portion of the sheet grip portion. As a consequence, when the extending portion 350 as the designated grip portion is moved to the outside of the device main body 3 in a plan view, the release sheet 304 can be released along the first portion X1 of the adhesion sheet 2 which is located below the support portion 24. For this reason, similar to the release sheet 4 (refer to Fig. 1 and the like) described above, the winding and bending of the second portion X2 of the adhesion sheet 2 caused by the release operation of the release sheet 304 can be further suppressed.

As illustrated in Figs. 23 and 24, in the present embodiment, the extending portions 350 of the two release sheets 304 of the release sheet group 380a may be moved in the direction of a white arrow "M1". In addition, as illustrated in Fig. 24, in the present embodiment, the extending portions 350 of the two release sheets 304 of the release sheet group 380b may be moved in the direction of a white arrow "M2".

In addition, as illustrated in Fig. 23 and the like, in the present embodiment, in a plan view, the extending portion 350 as the designated grip portion of the sheet grip portion is formed at a position different from that of the receiving portion 14 defined by the adhesion sheet 2. As a consequence, in a state where a portion of the insertion device such as a sheath which is inserted into the living body remains received by the receiving portion 14, the portion extending outside the living body, the extending portion 350 as the designated grip portion of the sheet grip portion can be operated to release the release sheet 304 from the adhesion sheet 2.

Further, as illustrated in Fig. 23 and the like, in a plan view, the release sheet 304 of the present embodiment is not provided in at least a part of the receiving portion 14 defined by the adhesion sheet 2. Namely, even in a pre-use state before the release sheet 304 is released, the receiving portion 14 defined by the adhesion sheet 2 can receive the insertion device 100 such as a sheath (refer to Fig. 10A and the like) from the outside.

### [Fifth embodiment]

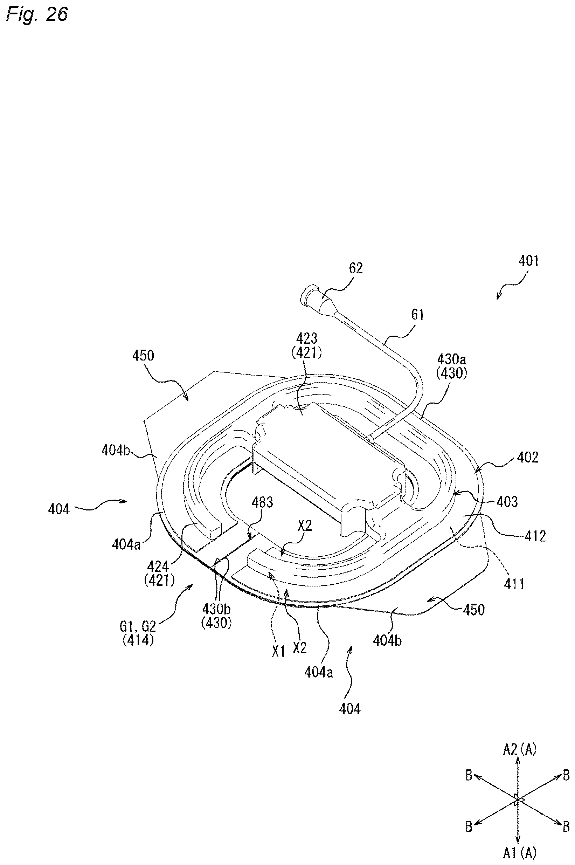

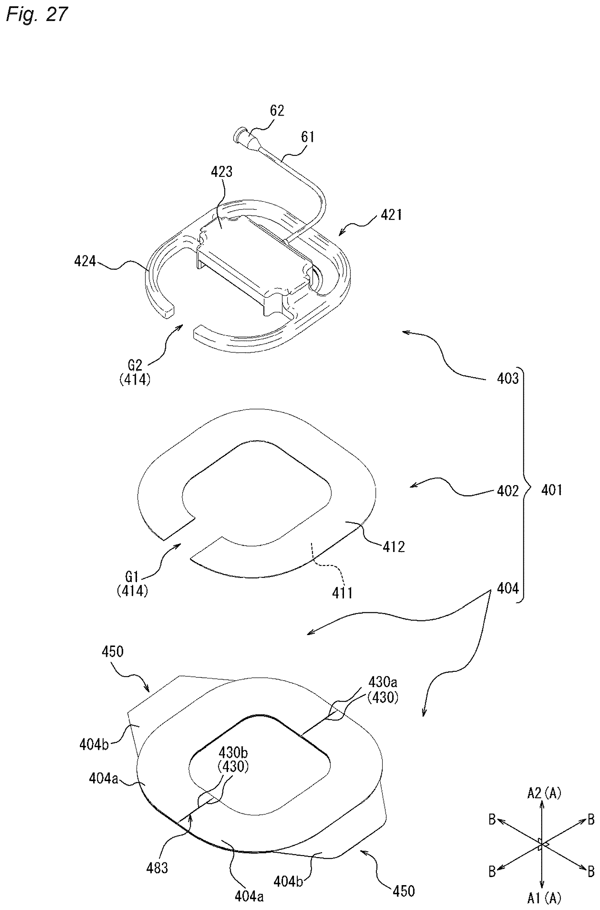

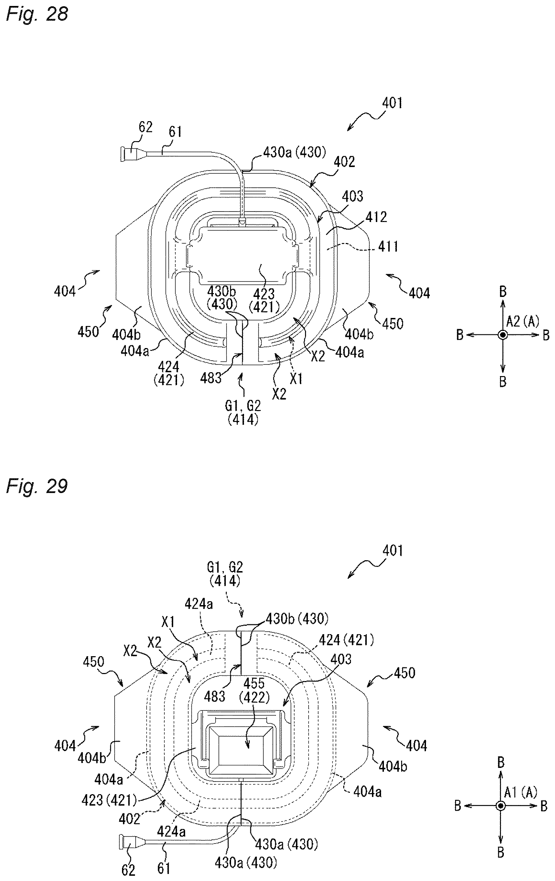

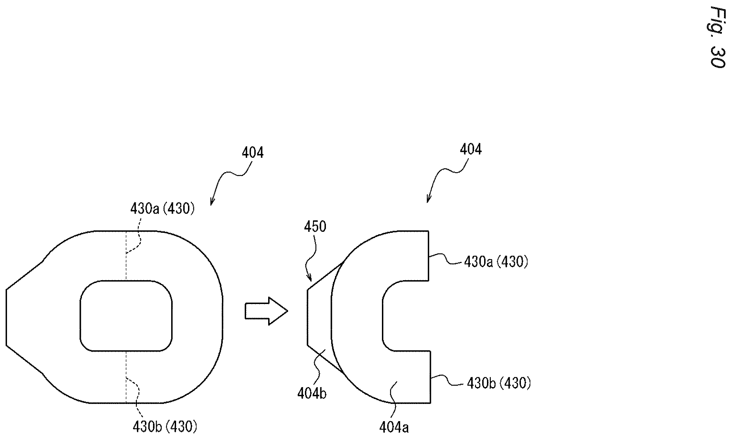

Next, an adhesion device 401 in a fifth embodiment will be described with reference to Figs. 26 to 30. Fig. 26 is a perspective view of the adhesion device 401. Fig. 27 is an exploded perspective view of the adhesion device 401. Fig. 28 is a top view of the adhesion device 401. Fig. 29 is a bottom view of the adhesion device 401. Fig. 30 is a view illustrating a release sheet 404 of the adhesion device 401. Fig. 30 illustrates a method of forming the release sheet 404.

The adhesion device 401 includes an adhesion sheet 402, a device main body 403, and the release sheet 404.

The adhesion sheet 402 is slightly different in shape in a plan view from the adhesion sheet 2 of the first embodiment, and other configurations are the same. Meanwhile, the shape of the device main body 403 to be described later is different from the shape of the device main body 3 of the first embodiment. For this reason, the positional relationship in a plan view between the adhesion sheet 402 and the device main body 403 of the present embodiment is different from the positional relationship in a plan view between the adhesion sheet 2 and the device main body 3 of the first embodiment. As illustrated in Fig. 28 and the like, for example, in a plan view, the adhesion sheet 402 of the present embodiment extends to surround a cover portion 423 of a holder 421 to be described later. In addition, as illustrated in Fig. 28 and the like, in a plan view, a gap G1 leading to the outside is formed in the adhesion sheet 402 at the position of a part of the periphery surrounding the cover portion 423 of the holder 421 to be described later.

The device main body 403 is mounted to a mounting surface 412 of the adhesion sheet 402. The device main body 403 includes the holder 421 and a compression member 422. Similar to the compression member 22 of the first embodiment, the compression member 422 of the present embodiment is an inflator 455. The shape of the holder 421 of the device main body 403 is different from that of the device main body 3 of the first embodiment. Particularly, as illustrated in Fig. 28, in a plan view, a support portion 424 of the holder 421 of the present embodiment does not fully surround the periphery of the cover portion 423. As illustrated in Fig. 28 and the like, in a plan view, a gap G2 leading to the outside is formed in the support portion 424 at the position of a part of the periphery surrounding the cover portion 423. The gap G2 is provided at the same position as that of the gap G1 of the adhesion sheet 402 having a C shape in a plan view. Therefore, in the adhesion device 401 of the present embodiment, a receiving portion 414 which can receive the insertion device 100 such as a sheath (refer to Fig. 10A and the like) is formed of the gap G2 of the support portion 424 of the holder 421 and the gap G1 of the adhesion sheet 402.

Further, the receiving portion 414 of the present embodiment extends to a central opening region defined by the adhesion sheet 402 having a C shape. Therefore, in the present embodiment, the insertion device 100 such as a sheath (refer to Fig. 10A and the like) can be received at the position of the central opening region defined by the adhesion sheet 402 having a C shape.

The release sheet 404 releasably adheres to an adhesion surface 411 of the adhesion sheet 402. When the adhesion device 401 adheres to the biological surface, the release sheet 404 is released from the adhesion surface 411. Accordingly, the adhesion surface 411 of the adhesion sheet 402 is exposed to the outside, and the adhesion device 401 is in the state of being able to adhere to the biological surface.

As illustrated in Figs. 26 to 30, the release sheet 404 includes a release layer 404a that adheres to the adhesion surface 411 of the adhesion sheet 402, and a folded layer 404b which is formed of at least one layer (one layer in the present embodiment) and is folded to a side, which is opposite to the adhesion surface 411 with respect to the release layer 404a, to be layered.

As illustrated in Fig. 26, the folded layer 404b of the release sheet 404 is layered on the release layer 404a of the release sheet 404 at least at the position of the second portion X2 of the adhesion sheet 402. Since the release sheet 404 has such a configuration, similar to the release sheet 4 of the first embodiment, the release sheet 404 is easily released from the second portion X2 of the adhesion sheet 402.

As illustrated in Fig. 27 and the like, in the present embodiment, the entire region of the adhesion surface 411 of the adhesion sheet 402 is covered with two release sheets 404 which are symmetrical in shape. Fig. 30 illustrates only one release sheet 404 of the two release sheets 404 which are symmetrical in shape. Since the two release sheets 404 are symmetrical in shape, hereinafter, only the one release sheet 404 will be described as an example. As illustrated in Fig. 30, the release sheet 404 of the present embodiment is folded once from an original form of a single layer (left figure in Fig. 30) to be shaped into a completed form of two layers (right figure in Fig. 30). In such a manner, the release sheet 404 including the release layer 404a which is formed of one layer and the folded layer 404b which is formed of one layer is formed.

The release sheet 404 of the present embodiment has two folds 430 between the release layer 404a and the folded layer 404b. The two folds 430 of the present embodiment are located on the same virtual straight line. Hereinafter, for convenience of description, the two folds 430 are referred as a "first fold 430a" and a "second fold 430b".

As illustrated in Fig. 29, in a plan view, the first fold 430a passes through the first portion X1 of the adhesion sheet 402. As a consequence, similar to the release sheet 4 (refer to Fig. 1 and the like) described above, the position of the first portion X1 of the adhesion sheet 402 can be set as a release start position of the release layer 404a of the release sheet 404. Accordingly, similar to the release sheet 4 (refer to Fig. 1 and the like) described above, the winding and bending of the second portion X2 of the adhesion sheet 402 caused by the release operation of the release sheet 404 can be suppressed.

As illustrated in Fig. 29, in a plan view, the second fold 430b does not pass through the first portion X1 of the adhesion sheet 402. However, in a plan view, the second fold 430b does not pass through the first portion X1 and the second portion X2 of the adhesion sheet 402. In addition, in a plan view, the second portion X2 does not intervene between the second fold 430b and the first portion X1 of the adhesion sheet 402. Therefore, the same effect as that in the first fold 430a can be obtained even at the position of the second fold 430b.

Hereinafter, items common to the first fold 430a and the second fold 430b will be described. For this reason, unless otherwise specified, the first fold 430a and the second fold 430b are simply referred to as the fold 430 without distinction.

In the release sheet 304, attention is paid to the folded layer 404b and the fold 430. As illustrated in Fig. 28 and the like, in a plan view, the device main body 403 is interposed between the fold 430 and an extending portion 450 as a designated grip portion of a sheet grip portion which is formed of the folded layer 404b. With such a configuration, similar to the release sheet 4 (refer to Fig. 1 and the like) described above, the release sheet 404 is easily released while passing through the first portion X1 of the adhesion sheet 402 on a lower surface of the device main body 403. Namely, similar to the release sheet 4 (refer to Fig. 1 and the like) described above, the winding and bending of the second portion X2 of the adhesion sheet 402 caused by the release operation of the release sheet 404 can be suppressed.

More specifically, as illustrated in Fig. 28 and the like, in a plan view, the holder 421 of the device main body 403, more specifically, the support portion 424 of the holder 421 is interposed between the fold 430 and the extending portion 450 as the designated grip portion of the sheet grip portion which is formed of the folded layer 404b. As illustrated in Fig. 28 and the like, in a plan view, the support portion 424 of the present embodiment extends to surround the cover portion 423. In addition, as illustrated in Fig. 29 and the like, in a plan view, a part of the support portion 424 (for example, refer to the position of reference sign "424a" in Fig. 29) of the present embodiment extends from the fold 430 in a direction toward the extending portion 450 as the designated grip portion of the sheet grip portion. As a consequence, when the extending portion 450 as the designated grip portion is moved to the outside of the device main body 403 in a plan view, the release sheet 404 can be released along the first portion X1 of the adhesion sheet 402 which is located below the support portion 424. For this reason, similar to the release sheet 4 (refer to Fig. 1 and the like) described above, the winding and bending of the second portion X2 of the adhesion sheet 402 caused by the release operation of the release sheet 404 can be further suppressed.

In addition, as illustrated in Fig. 28 and the like, in the present embodiment, in a plan view, the extending portion 450 as the designated grip portion of the sheet grip portion is formed at a position different from that of the receiving portion 414 defined by the adhesion sheet 402 and the support portion 424. As a consequence, in a state where a portion of the insertion device such as a sheath which is inserted into the living body remains received by the receiving portion 414, the portion extending outside the living body, the extending portion 450 as the designated grip portion of the sheet grip portion can be operated to release the release sheet 404 from the adhesion sheet 402.

Further, as illustrated in Fig. 28 and the like, in a plan view, the release sheet 404 of the present embodiment is located in the gaps G1 and G2 described above. Namely, in a plan view, the release sheet 404 of the present embodiment closes the receiving portion 414 defined by the adhesion sheet 402 and the support portion 424. However, the release sheet 404 has a split 483 at the position of the gaps G1 and G2 as the receiving portion 414. More specifically, two release sheets 404 of the present embodiment are divided at the position of the receiving portion 414. Namely, the release sheet 404 of the present embodiment has a discontinuous configuration at the position of the receiving portion 414. For this reason, even in a pre-use state before the release sheet 404 is released, the receiving portion 414 defined by the adhesion sheet 402 and the support portion 424 can receive the insertion device 100 such as a sheath (refer to Fig. 10A and the like) from the outside through the split 483 of the release sheet 404.

The adhesion device according to the present disclosure is not limited to the specific configurations described in the embodiments described above, and various modifications and changes can be made without departing from the concept of the present disclosure. For example, an adhesion device configured by appropriately combining the components illustrated in the first to fifth embodiments described above also belongs to the technical scope of the present disclosure.

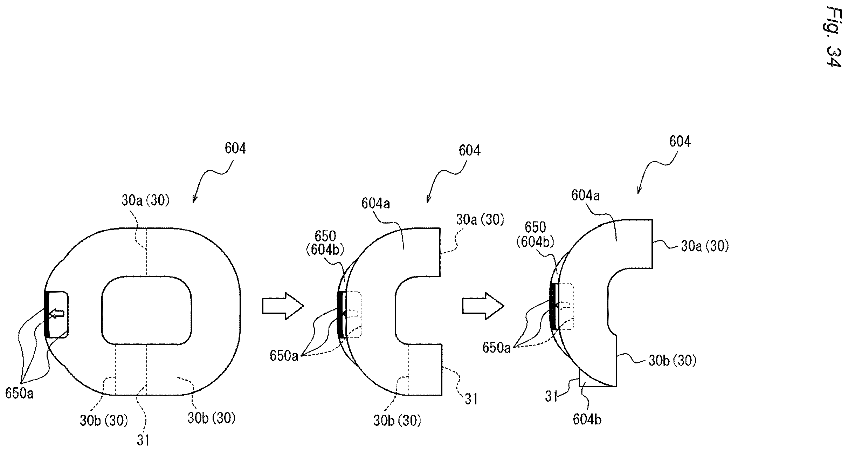

Fig. 34 is a view illustrating a release sheet 604 as a modification example of the release sheet 4 illustrated in Fig. 5. Fig. 34 illustrates a method of forming the release sheet 604. The configuration of a designated grip portion of the release sheet 604 illustrated in Fig. 34 is different from that of the release sheet 4 illustrated in Fig. 5, and other configurations are the same. The designated grip portion in a folded layer 604b as a sheet grip portion of the release sheet 604 illustrated in Fig. 34 is configured to serve as both the same extending portion as the extending portion 50 illustrated in Fig. 5 and the same grip guide portion as the grip guide portion 550 illustrated in Fig. 33. Hereinafter, for convenience of description, the designated grip portion illustrated in Fig. 34 is referred to as an "extending grip guide portion 650". The extending grip guide portion 650 is formed of a portion that does not overlap a release layer 604a, and has a predetermined mark 650a which provides a guide on gripping. Examples of the mark 650a of the extending grip guide portion 650 illustrated in Fig. 34 include various symbols, colors, patterns, characters, combinations thereof, and the like which provide a guide on gripping. In Fig. 34, as one example, a guide on gripping is provided by indicating the grip range with a line and the grip position with a color, and when the release sheet 604 is released, a direction in which the extending grip guide portion 650 is moved is guided by an arrow.

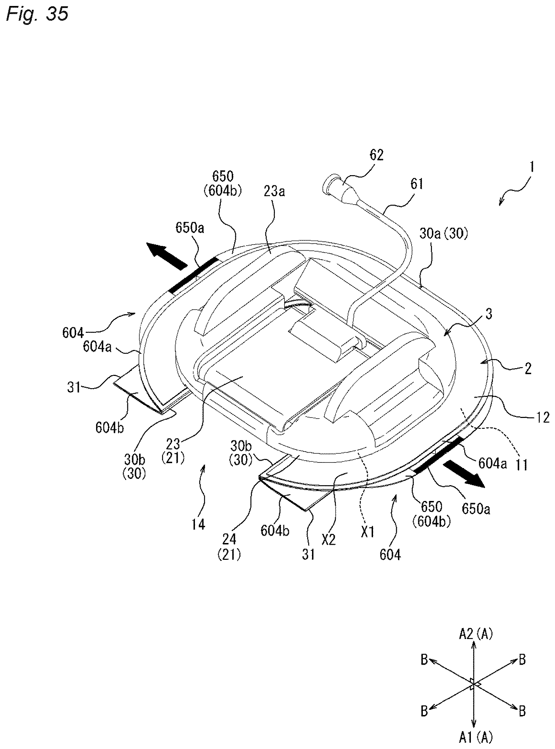

Fig. 35 is a perspective view illustrating a state where instead of the release sheet 4 illustrated in Fig. 5, the release sheet 604 illustrated in Fig. 34 adheres to the adhesion surface 11 of the adhesion sheet 2. An operator such as a health care worker grips the extending grip guide portion 650 and moves the extending grip guide portion 650 in a direction indicated by a thick arrow in Fig. 35. Accordingly, the release sheet 604 can be released from the adhesion surface 11 of the adhesion sheet 2. As illustrated in Figs. 34 and 35, the extending grip guide portion 650 serving as the extending portion and the grip guide portion is set as the designated grip portion, the operator can easily and intuitively understand the grip position, the operation method, and the like.

### Industrial Applicability

The present disclosure relates to the adhesion device.

### Reference Signs List

1, 101, 201, 301, 401: adhesion device
2, 402: adhesion sheet
3, 403: device main body
4, 104, 204, 304, 404, 504, 604: release sheet
4a, 104a, 204a, 304a, 404a, 504a, 604a: release layer
4b, 104b, 204b, 304b, 404b, 504b, 604b: folded layer (sheet grip portion)
11, 411: adhesion surface
12, 412: mounting surface
13: outer peripheral portion of adhesion sheet
14: receiving portion
21, 421: holder
22, 422: compression member
22a: contact portion
22b: pressing portion
23, 423: cover portion
23a: plate portion
24, 424: support portion
24a, 424a: part of support portion
30, 230, 330, 430: fold
30a: first fold
30b: second fold
31: fold
50, 250, 350, 450: extending portion (designated grip portion)
55, 455: inflator
55a: internal space of inflator
60a: first inflator
60a1: internal space of first inflator
60b: second inflator
60b1: internal space of second inflator
61: tube
62: connection section
70: syringe
100: insertion device
204a1: projection portion
204b1: slit
380a, 380b: release sheet group
430a: first fold
430b: second fold
483: split of release sheet
550: grip guide portion (designated grip portion)
550a, 650a: mark
650: extending grip guide portion (designated grip portion)
A: thickness direction of adhesion sheet
A1: downward direction
A2: upward direction
B: extending direction of adhesion sheet
G1, G2: gap
M1, M2: movement direction of extending portion
P: perforation
X1: first portion of adhesion sheet
X2: second portion of adhesion sheet
BS: biological surface
CT: connective tissue
FV: femoral vein

## Claims

1. A medical adhesion device (1, 101, 201, 301, 401) comprising:
an adhesion sheet (2, 402) including an adhesion surface (11, 411) that is adherable to a biological surface (BS), and a mounting surface (12, 412) that is located on a side opposite to the adhesion surface (11, 411);
a device main body (3, 403) that is mounted to the mounting surface (12, 412) of the adhesion sheet (2, 402); and
a release sheet (4, 104, 204, 304, 404, 504, 604) that releasably adheres to the adhesion surface (11, 411) of the adhesion sheet (2, 402),
wherein the adhesion sheet (2, 402) includes
a first portion to which the device main body (3, 403) is mounted, and
a second portion to which the device main body (3, 403) is not mounted,
the release sheet (4, 104, 204, 304, 404, 504, 604) includes
a release layer (4a, 104a, 204a, 304a, 404a, 504a, 604a) that adheres to the adhesion surface (11, 411) of the adhesion sheet (2, 402), and
a folded layer (4b, 104b, 204b, 304b, 404b, 504b, 604b) which is formed of at least one layer and is folded to a side, which is opposite to the adhesion surface (11, 411) with respect to the release layer (4a, 104a, 204a, 304a, 404a, 504a, 604a), to be layered, and
the folded layer (4b, 104b, 204b, 304b, 404b, 504b, 604b) of the release sheet (4, 104, 204, 304, 404, 504, 604) is layered on the release layer (4a, 104a, 204a, 304a, 404a, 504a, 604a) of the release sheet (4, 104, 204, 304, 404, 504, 604) at least at a position of the second portion of the adhesion sheet (2, 402), **characterized in that** the folded layer (4b, 104b, 204b, 304b, 404b, 504b, 604b) of the release sheet (4, 104, 204, 304, 404, 504, 604) forms a sheet grip portion that is gripped when the release sheet (4, 104, 204, 304, 404, 504, 604) is released from the adhesion sheet (2, 402),
the sheet grip portion includes a designated grip portion (550,650), and
the designated grip portion (550, 650) includes at least one of an extending portion which is formed of a portion of the folded layer (4b, 104b, 204b, 304b, 404b, 504b, 604b) of the release sheet (4, 104, 204, 304, 404, 504, 604), the portion not overlapping the release layer (4a, 104a, 204a, 304a, 404a, 504a, 604a) in a plan view seen along a thickness direction (A2) of the adhesion sheet (2, 402), and a grip guide portion which is a portion of the folded layer (4b, 104b, 204b, 304b, 404b, 504b, 604b) of the release sheet (4, 104, 204, 304, 404, 504, 604, the portion overlapping the release layer (4a, 104a, 204a, 304a, 404a, 504a, 604a in the plan view seen along the thickness direction of the adhesion sheet, and to which a predetermined mark (550a, 650a) which provides a guide on gripping is applied,
in the plan view seen along the thickness direction (A2), at least one fold (30, 31, 230, 330, 430) between the release layer (4a, 104a, 204a, 304a, 404a, 504a, 604a) and the folded layer (4b, 104b, 204b, 304b, 404b, 504b, 604b) of the release sheet (4, 104, 204, 304, 404, 504, 604) passes through the first portion of the adhesion sheet (2, 402),
in the plan view seen along the thickness direction (A2), the device main body (3, 403) is interposed between a portion of the at least one fold (30, 31, 230, 330, 430) and the designated grip portion (550,650) of the sheet grip portion, the portion overlapping the first portion of the adhesion sheet (2, 402),
the device main body (3, 403) includes
a holder (21, 421) that is fixed to the mounting surface (12, 412) of the adhesion sheet (2, 402), and
a compression member (22, 422) that is held by the holder (21, 421) and protrudes or is protrudable toward one side in the thickness direction from the adhesion surface (11, 411) of the adhesion sheet (2, 402) to compress the biological surface (BS), and
in the plan view seen along the thickness direction (A2), the holder (21, 421) is interposed between the portion of the at least one fold (30, 31, 230, 330, 430) and the designated grip portion (550,650) of the sheet grip portion, the portion overlapping the first portion of the adhesion sheet (2, 402),
the adhesion sheet (2, 402) includes an outer peripheral portion that is located on a periphery outside the device main body (3, 403) in the plan view seen along the thickness direction (A2),
in the plan view seen along the thickness direction (A2), the outer peripheral portion of the adhesion sheet (2, 402) does not fully surround the periphery of the device main body (3, 403), and a receiving portion (14) configured to receive an insertion device (100) is defined around the device main body (3, 403) at a position where the outer peripheral portion of the adhesion sheet (2, 402) is not provided, and
in the plan view seen along the thickness direction (A2) the designated grip portion (550, 650) of the sheet grip portion is formed at a position different from a position of the receiving portion (14).

2. The adhesion device according to claim 1,
wherein the holder (21, 421) includes
a cover portion (23, 423) that covers the other side of the compression member (22, 422) in the thickness direction, and
a support portion (24, 424) that supports the cover portion (23, 423), and is fixed to the mounting surface (12, 412) of the adhesion sheet (2, 402), and
in the plan view seen along the thickness direction (A2), the support portion (24, 424) of the holder (21, 421) is interposed between the portion of the at least one fold (30, 31, 230, 330, 430) and the designated grip portion of the sheet grip portion, the portion overlapping the first portion of the adhesion sheet (2, 402).

3. The medical adhesion device (1, 101, 201, 301, 401) according to claim 2,
wherein in the plan view seen along the thickness direction (A2), the support portion (24, 424) extends to surround the cover portion (23, 423), and
in the plan view seen along the thickness direction (A2), a part of the support portion (24, 424) extends from the portion of the fold (30, 31, 230, 330, 430) in a direction toward the designated grip portion (550, 650) of the sheet grip portion, the portion overlapping the first portion of the adhesion sheet (2, 402).

4. The medical adhesion device (1, 101, 201, 301, 401) according to claim 1,
wherein in the plan view seen along the thickness direction (A2), the release sheet (4, 104, 204, 304, 404, 504, 604) is not provided in at least a part of the receiving portion (14).

5. The medical adhesion device (1, 101, 201, 301, 401) according to claim 3,
wherein in the plan view seen along the thickness direction (A2), the adhesion sheet (2, 402) extends to surround the cover portion (23, 423),
a gap (G1, G2) leading to an outside is formed in the adhesion sheet (2, 402) and the support portion (24, 424) at the same position of a part of a periphery surrounding the cover portion (23, 423) in the plan view seen along the thickness direction (A2), and
a receiving portion (14) configured to receive an insertion device (100) is formed of the gap (G1, G2).

6. The medical adhesion device (1, 101, 201, 301, 401) according to claim 5,
wherein in the plan view seen along the thickness direction (A2), the release sheet (4, 104, 204, 304, 404, 504, 604) is located at the gap (G1, G2), and has a split at a position of the gap (G1, G2).

## Patentansprüche

1. Medizinische Adhäsionsvorrichtung (1, 101, 201, 301, 401), umfassend:
eine Adhäsionsfolie (2, 402), die eine Adhäsionsfläche (11, 411), welche an einer biologischen Oberfläche (BS) anhaftbar ist, und eine Befestigungsfläche (12, 412) umfasst, die auf einer Seite angeordnet ist, welche der Adhäsionsfläche (11, 411) gegenüberliegt;
einen Vorrichtungshauptkörper (3, 403), der an der Befestigungsfläche (12, 412) der Adhäsionsfolie (2, 402) befestigt ist; und
eine Trennfolie (4, 104, 204, 304, 404, 504, 604), die ablösbar an der Adhäsionsfläche (11, 411) der Adhäsionsfolie (2, 402) anhaftet,
wobei die Adhäsionsfolie (2, 402) umfasst
einen ersten Abschnitt, an dem der Vorrichtungshauptkörper (3, 403) angebracht ist, und
einen zweiten Abschnitt, an dem der Vorrichtungshauptkörper (3, 403) nicht angebracht ist, und
wobei die Trennfolie (4, 104, 204, 304, 404, 504, 604) umfasst
eine Trennschicht (4a, 104a, 204a, 304a, 404a, 504a, 604a), die an der Adhäsionsfläche (11, 411) der Adhäsionsfolie (2, 402) anhaftet, und
eine gefaltete Lage (4b, 104b, 204b, 304b, 404b, 504b, 604b), die aus mindestens einer Lage gebildet wird und auf eine Seite gefaltet ist, die in Bezug auf die Trennschicht (4a, 104a, 204a, 304a, 404a, 504a, 604a) der Adhäsionsfläche (11, 411) gegenüberliegt, auf der sie aufgelegt wird, und
die gefaltete Lage (4b, 104b, 204b, 304b, 404b, 504b, 604b) der Trennfolie (4, 104, 204, 304, 404, 504, 604) auf die Trennschicht (4a, 104a, 204a, 304a, 404a, 504a, 604a) der Trennfolie (4, 104, 204, 304, 404, 504, 604) mindestens an einer Position des zweiten Abschnitts der Adhäsionsfolie (2, 402) aufgelegt ist, **dadurch gekennzeichnet, dass** die gefaltete Lage (4b, 104b, 204b, 304b, 404b, 504b, 604b) der Trennfolie (4, 104, 204, 304, 404, 504, 604) einen Foliengriffabschnitt bildet, der gegriffen wird, wenn die Trennfolie (4, 104, 204, 304, 404, 504, 604) von der Adhäsionsfolie (2, 402) gelöst wird,
der Foliengriffabschnitt einen gekennzeichneten Griffabschnitt (550, 650) umfasst, und
der gekennzeichnete Griffabschnitt (550, 650) mindestens eines umfasst von
einem verlängerten Abschnitt, der aus einem Abschnitt der gefalteten Lage (4b, 104b, 204b, 304b, 404b, 504b, 604b) der Trennfolie (4, 104, 204, 304, 404, 504, 604) gebildet ist, der Abschnitt, der die Trennschicht (4a, 104a, 204a, 304a, 404a, 504a, 604a) in einer Draufsicht gesehen entlang einer Dickenrichtung (A2) der Adhäsionsfolie (2, 402) nicht überlappt, und einem Griff-Führungsabschnitt, der ein Abschnitt der gefalteten Lage (4b, 104b, 204b, 304b, 404b, 504b, 604b) der Trennfolie (4, 104, 204, 304, 404, 504, 604) ist, desjenigen Abschnitts, welcher die Trennschicht (4a, 104a, 204a, 304a, 404a, 504a, 604a) in der Draufsicht gesehen entlang der Dickenrichtung der Adhäsionsfolie überlappt und auf den eine vorbestimmte Markierung (550a, 650a) aufgebracht ist, die als Führung zum Greifen dient,
in der Draufsicht gesehen entlang der Dickenrichtung (A2) mindestens eine Falte (30, 31, 230, 330, 430) zwischen der Trennschicht (4a, 104a, 204a, 304a, 404a, 504a, 604a) und der gefalteten Schicht (4b, 104b, 204b, 304b, 404b, 504b, 604b) der Trennfolie (4, 104, 204, 304, 404, 504, 604) durch den ersten Abschnitt der Adhäsionsfolie (2, 402) hindurchgeht,
in der Draufsicht entlang der Dickenrichtung (A2) gesehen, der Vorrichtungshauptkörper (3, 403) zwischen einem Abschnitt der mindestens einen Falte (30, 31, 230, 330, 430) und dem gekennzeichneten Griffabschnitt (550, 650) des Folien-Griffabschnitts, desjenigen Abschnitts, welcher den ersten Abschnitt des Adhäsionsabschnitts (2, 402) überlappt, angeordnet ist,
wobei der Vorrichtungshauptkörper (3, 403) umfasst
eine Halterung (21, 421), die an der Befestigungsfläche (12, 412) der Adhäsionsfolie (2, 402) befestigt ist, und
ein Kompressionselement (22, 422), das von der Halterung (21, 421) gehalten wird und in Richtung einer Seite in der Dickenrichtung von der Adhäsionsfläche (11, 411) der Adhäsionsfolie (2, 402) hervorsteht oder hervorstehbar ist, um die biologische Oberfläche (BS) zusammenzudrücken, und
in der Draufsicht entlang der Dickenrichtung (A2) gesehen, ist die Halterung (21, 421) zwischen dem Abschnitt der mindestens einen Falte (30, 31, 230, 330, 430) und dem gekennzeichneten Griffabschnitt (550, 650) des Folien-Griffabschnitts angeordnet, desjenigen Abschnitts, welcher den ersten Abschnitt des Adhäsionsabschnitts (2, 402) überlappt,
die Adhäsionsfolie (2, 402) einen äußeren Umfangsabschnitt aufweist, der an einem Umfang außerhalb des Vorrichtungshauptkörpers (3, 403) in der Draufsicht entlang der Dickenrichtung (A2) gesehen angeordnet ist, in der Draufsicht entlang der Dickenrichtung (A2) gesehen, der äußere Umfangsabschnitt der Adhäsionsfolie (2, 402) den Umfang des Vorrichtungshauptkörpers (3, 403) nicht vollständig umgibt, und ein Aufnahmeabschnitt (14), der so konfiguriert ist, dass er eine Einführvorrichtung (100) aufnimmt, ist um den Vorrichtungshauptkörper (3, 403) herum an einer Position definiert, an welcher der äußere Umfangsabschnitt der Adhäsionsfolie (2, 402) nicht vorgesehen ist, und
in der Draufsicht entlang der Dickenrichtung (A2)gesehen, ist der gekennzeichnete Griffabschnitt (550, 650) des Folien-Griffabschnitts an einer Position ausgebildet, die sich von der Position des Aufnahmeabschnitts (14) unterscheidet.

2. Adhäsionsvorrichtung nach Anspruch 1, wobei die Halterung (21, 421) umfasst
einen Abdeckabschnitt (23, 423), der die andere Seite des Kompressionselements (22, 422) in der Dickenrichtung abdeckt, und
einen Stützabschnitt (24, 424), der den Abdeckabschnitt (23, 423) stützt und an der Befestigungsfläche (12, 412) der Adhäsionsfolie (2, 402) befestigt ist, und
in der Draufsicht entlang der Dickenrichtung (A2) gesehen, ist der Stützabschnitt (24, 424) der Halterung (21, 421) zwischen dem Abschnitt der mindestens einen Falte (30, 31, 230, 330, 430) und dem gekennzeichneten Griffabschnitt (550, 650) des Folien-Griffabschnitts, desjenigen Abschnitts, welcher den ersten Abschnitt des Adhäsionsabschnitts (2, 402) überlappt, angeordnet.

3. Medizinische Adhäsionsvorrichtung (1, 101, 201, 301, 401) nach Anspruch 2,
wobei sich in der Draufsicht entlang der Dickenrichtung (A2) gesehen der Stützabschnitt (24, 424) so erstreckt, dass er den Abdeckabschnitt (23, 423) umgibt, und
in der Draufsicht entlang der Dickenrichtung (A2) gesehen, ein Teil des Stützabschnitts (24, 424) sich von dem Abschnitt der Falte (30, 31, 230, 330, 430) in einer Richtung zu dem gekennzeichneten Griffabschnitt (550, 650) des Folien-Griffabschnitts, des Abschnitts, der den ersten Abschnitt des Adhäsionsabschnitts (2, 402) überlappt, erstreckt.

4. Medizinische Adhäsionsvorrichtung (1, 101, 201, 301, 401) nach Anspruch 1,
wobei in der Draufsicht entlang der Dickenrichtung (A2) gesehen, die Trennfolie (4, 104, 204, 304, 404, 504, 604) mindestens in einem Teil des Aufnahmeabschnitts (14) nicht vorgesehen ist.

5. Medizinische Adhäsionsvorrichtung (1, 101, 201, 301, 401) nach Anspruch 3,
wobei in der Draufsicht entlang der Dickenrichtung (A2) gesehen, die Adhäsionsfolie (2, 402) sich so erstreckt, dass sie den Abdeckabschnitt (23, 423) umgibt,
ein Spalt (G1, G2), der zu einer Außenseite führt, in der Adhäsionsfolie (2, 402) und dem Stützabschnitt (24, 424) an derselben Position von einem Teil eines Umfangs, der den Abdeckabschnitt (23, 423) in der Draufsicht entlang der Dickenrichtung (A2) gesehen, umgibt, ausgebildet ist, und
ein Aufnahmeabschnitt (14), der zum Aufnehmen einer Einführvorrichtung (100) konfiguriert ist, aus dem Spalt (G1, G2) ausgebildet ist.

6. Medizinische Adhäsionsvorrichtung (1, 101, 201, 301, 401) nach Anspruch 5,
wobei in der Draufsicht entlang der Dickenrichtung (A2) gesehen, die Trennfolie (4, 104, 204, 304, 404, 504, 604) an dem Spalt (G1, G2) vorgesehen ist und an einer Position des Spaltes (G1, G2) eine Aufteilung aufweist.

## Revendications

1. Dispositif adhésif médical (1, 101, 201, 301, 401) comprenant :
une feuille adhésive (2, 402) incluant une surface adhésive (11, 411) qui peut adhérer à une surface biologique (BS), et une surface de montage (12, 412) qui est située sur un côté opposé à la surface adhésive (11, 411) ;
un corps principal de dispositif (3, 403) qui est monté sur la surface de montage (12, 412) de la feuille adhésive (2, 402) ; et
une feuille antiadhésive (4, 104, 204, 304, 404, 504, 604) qui adhère de manière libérable à la surface adhésive (11, 411) de la feuille adhésive (2, 402),
dans lequel la feuille adhésive (2, 402) inclut
une première partie sur laquelle le corps principal de dispositif (3, 403) est monté, et
une seconde partie sur laquelle le corps principal de dispositif (3, 403) n'est pas monté,
la feuille antiadhésive (4, 104, 204, 304, 404, 504, 604) inclut
une couche antiadhésive (4a, 104a, 204a, 304a, 404a, 504a, 604a) qui adhère à la surface adhésive (11, 411) de la feuille adhésive (2, 402), et
une couche pliée (4b, 104b, 204b, 304b, 404b, 504b, 604b) qui est formée d'au moins une couche et est pliée sur un côté, qui est opposé à la surface adhésive (11, 411) par rapport à la couche antiadhésive (4a, 104a, 204a, 304a, 404a, 504a, 604a), à superposer, et
la couche pliée (4b, 104b, 204b, 304b, 404b, 504b, 604b) de la feuille antiadhésive (4, 104, 204, 304, 404, 504, 604) est superposée sur la couche antiadhésive (4a, 104a, 204a, 304a, 404a, 504a, 604a) de la feuille antiadhésive (4, 104, 204, 304, 404, 504, 604) au moins en une position de la seconde partie de la feuille adhésive (2, 402), **caractérisé en ce que** la couche pliée (4b, 104b, 204b, 304b, 404b, 504b, 604b) de la feuille antiadhésive (4, 104, 204, 304, 404, 504, 604) forme une partie de préhension de feuille qui est saisie lorsque la feuille antiadhésive (4, 104, 204, 304, 404, 504, 604) est décollée de la feuille adhésive (2, 402),
la partie de préhension de feuille inclut une partie de préhension désignée (550, 650), et
la partie de préhension désignée (550, 650) inclut au moins l'une parmi une partie d'extension qui est formée d'une partie de la couche pliée (4b, 104b, 204b, 304b, 404b, 504b, 604b) de la feuille antiadhésive (4, 104, 204, 304, 404, 504, 604), la partie ne chevauchant pas la couche antiadhésive (4a, 104a, 204a, 304a, 404a, 504a, 604a) dans une vue en plan observée le long du sens de l'épaisseur (A2) de la feuille adhésive (2, 402), et une partie de guidage de préhension qui est une partie de la couche pliée (4b, 104b, 204b, 304b, 404b, 504b, 604b) de la feuille antiadhésive (4, 104, 204, 304, 404, 504, 604), la partie chevauchant la couche antiadhésive (4a, 104a, 204a, 304a, 404a, 504a, 604a) dans la vue en plan observée le long du sens de l'épaisseur de la feuille adhésive, et sur laquelle est appliquée une marque prédéterminée (550a, 650a) qui sert de guide lors de la préhension,
dans la vue en plan observée le long du sens de l'épaisseur (A2), au moins un pli (30, 31, 230, 330, 430) entre la couche antiadhésive (4a, 104a, 204a, 304a, 404a, 504a, 604a) et la couche pliée (4b, 104b, 204b, 304b, 404b, 504b, 604b) de la feuille antiadhésive (4, 104, 204, 304, 404, 504, 604) passe à travers la première partie de la feuille adhésive (2, 402),
dans la vue en plan observée le long du sens de l'épaisseur (A2), le corps principal de dispositif (3, 403) est interposé entre une partie de l'au moins un pli (30, 31, 230, 330, 430) et la partie de préhension désignée (550, 650) de la partie de préhension de feuille, la partie chevauchant la première partie de la feuille adhésive (2, 402),
le corps principal de dispositif (3, 403) inclut
un élément de retenue (21, 421) qui est fixé à la surface de montage (12, 412) de la feuille adhésive (2, 402), et
un organe de compression (22, 422) qui est retenu par l'élément de retenue (21, 421) et fait saillie ou est apte à faire saillie vers un côté dans le sens de l'épaisseur à partir de la surface adhésive (11, 411) de la feuille adhésive (2, 402) pour comprimer la surface biologique (BS), et
dans la vue en plan observée le long du sens de l'épaisseur (A2), l'élément de retenue (21, 421) est interposé entre la partie de l'au moins un pli (30, 31, 230, 330, 430) et la partie de préhension désignée (550, 650) de la partie de préhension de feuille, la partie chevauchant la première partie de la feuille adhésive (2, 402),
la feuille adhésive (2, 402) inclut une partie périphérique externe qui est située sur une périphérie à l'extérieur du corps principal de dispositif (3, 403) dans la vue en plan observée le long du sens de l'épaisseur (A2),
dans la vue en plan observée le long du sens de l'épaisseur (A2), la partie périphérique externe de la feuille adhésive (2, 402) n'entoure pas entièrement la périphérie du corps principal de dispositif (3, 403), et une partie de réception (14) configurée pour recevoir un dispositif d'insertion (100) est définie autour du corps principal de dispositif (3, 403) en une position où la partie périphérique externe de la feuille adhésive (2, 402) n'est pas fournie, et
dans la vue en plan observée le long du sens de l'épaisseur (A2), la partie de préhension désignée (550, 650) de la partie de préhension de feuille est formée en une position différente d'une position de la partie de réception (14).

2. Dispositif adhésif selon la revendication 1,
dans lequel l'élément de retenue (21, 421) inclut
une partie de recouvrement (23, 423) qui recouvre l'autre côté de l'organe de compression (22, 422) dans le sens de l'épaisseur, et
une partie de support (24, 424) qui supporte la partie de recouvrement (23, 423), et est fixée à la surface de montage (12, 412) de la feuille adhésive (2, 402), et
dans la vue en plan observée le long du sens de l'épaisseur (A2), la partie de support (24, 424) de l'élément de retenue (21, 421) est interposée entre la partie de l'au moins un pli (30, 31, 230, 330, 430) et la partie de préhension désignée de la partie de préhension de feuille, la partie chevauchant la première partie de la feuille adhésive (2, 402).

3. Dispositif adhésif médical (1, 101, 201, 301, 401) selon la revendication 2,
dans lequel, dans la vue en plan observée le long du sens de l'épaisseur (A2), la partie de support (24, 424) s'étend pour entourer la partie de recouvrement (23, 423), et
dans la vue en plan observée le long du sens de l'épaisseur (A2), une partie de la partie de support (24, 424) s'étend à partir de la partie du pli (30, 31, 230, 330, 430) dans une direction allant vers la partie de préhension désignée (550, 650) de la partie de préhension de feuille, la partie chevauchant la première partie de la feuille adhésive (2, 402).

4. Dispositif adhésif médical (1, 101, 201, 301, 401) selon la revendication 1,
dans lequel, dans la vue en plan observée le long du sens de l'épaisseur (A2), la feuille antiadhésive (4, 104, 204, 304, 404, 504, 604) n'est pas disposée dans au moins une partie de la partie de réception (14).

5. Dispositif adhésif médical (1, 101, 201, 301, 401) selon la revendication 3,
dans lequel, dans la vue en plan observée le long du sens de l'épaisseur (A2), la feuille adhésive (2, 402) s'étend pour entourer la partie de recouvrement (23, 423),
un espace (G1, G2) menant à un extérieur est formé dans la feuille adhésive (2, 402) et la partie de support (24, 424) à la même position d'une partie d'une périphérie entourant la partie de recouvrement (23, 423) dans la vue en plan observée le long du sens de l'épaisseur (A2), et
une partie de réception (14) configurée pour recevoir un dispositif d'insertion (100) est formée de l'espace (G1, G2).

6. Dispositif adhésif médical (1, 101, 201, 301, 401) selon la revendication 5,
dans lequel, dans la vue en plan observée le long du sens de l'épaisseur (A2), la feuille antiadhésive (4, 104, 204, 304, 404, 504, 604) est située au niveau de l'espace (G1, G2), et présente une fente en une position de l'espace (G1, G2) .
